# FASCICULE DE BREVET EUROPEEN

(11) **EP 2 091 920 B1**
(45) Date de publication et mention de la délivrance du brevet: **22.10.2014**
(21) Numéro de dépôt: 07870263.6
(22) Date de dépôt: 09.11.2007
(51) Int. Cl.: C07D 231/38, A61K 31/415, A61P 35/00

(54) **PYRAZOLES SUBSTITUEES, COMPOSITIONS LES CONTENANT, PROCEDE DE FABRICATION ET UTILISATION**
SUBSTITUIERTE PYRAZOLE, ZUSAMMENSETZUNGEN DAMIT SOWIE VERFAHREN ZU IHRER HERSTELLUNG UND VERWENDUNG
SUBSTITUTED PYRAZOLES, COMPOSITIONS CONTAINING THESE, METHOD OF PRODUCTION AND USE

(30) Priorité: 10.11.2006 FR 0609812
(43) Date de publication de la demande: 26.08.2009
(73) Titulaire: SANOFI, 75008 Paris (FR)
(72) Inventeur: BJERGARDE, Kirsten, Tucson, AZ 85737-9525 (US); DODSON, Mark, Tucson, AZ 85737-9525 (US); MAUGER, Jacques, Tucson, AZ 85737-9525 (US); NAIR, Anil, Tucson, AZ 85737-9525 (US); PATEK, Marcel, Tucson, AZ 85737-9525 (US); TABART, Michel, 75013 Paris (FR)
(74) Mandataire: Holubec, Méhdi
(86) Numéro de dépôt international: PCT/FR2007/001851
(87) Numéro de publication internationale: WO 2008/065282

(56) Documents cités:
- EP-A- 1 683 796
- WO-A-02/088090
- WO-A-2004/094410
- WO-A-2006/003440
- WO-A-2006/018662
- WO-A-2006/070198
- WO-A-2006/077414
- WO-A-2006/077425
- WO-A1-2005/012256
- US-A1- 2004 220 186
- J.L LARS AND CO: "Studies on carbanilic acidesters of cyclic amino alcohols.4-esters of pyrrolidinols and piperidinols as local anesthetics" JOURNAL OF MEDICINAL CHEMISTRY, vol. 14, no. 8, 1971, pages 710-714, XP002479633

## Description

La présente invention concerne notamment de nouveaux composés chimiques, particulièrement des pyrazoles substitués, les compositions les contenant, et leur utilisation comme médicaments.

Plus particulièrement, et selon un premier aspect, l'invention concerne de nouveaux pyrazoles substitués spécifiques présentant une activité anticancéreuse, via la modulation de l'activité de protéines, en particulier des kinases.

Les protéines kinases sont une famille d'enzymes qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

WO 2005/012256 décrit des pyrazoles disubstitués et leurs utilisations comme inhibiteurs des kinases CDKs et GSK-3.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, on peut citer KDR, Tie2, VEGFR-1, PDGFR, FGFR, FLT1. Les kinases KDR et/ou Tie2 sont préférées.

Ces produits répondent à la formule générale (I) suivante : 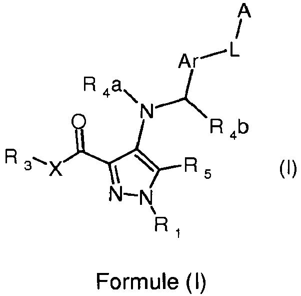 dans laquelle :
1) A et Ar sont indépendamment sélectionnés dans le groupe constitué par : aryle, hétéroaryle, aryle substitué, hétéroaryle substitué;
2) L est sélectionné dans le groupe constitué par: NH-CO-NH et O-CO-NH;
3) R₁ est sélectionné dans le groupe constitué par : H, R₆, COR₆, SO₂R₆, dans lequel R₆ est choisi parmi H, OR₇, NR₈R₉, alkyle, cycloalkyle, hétérocyclyle, hétérocyclyle substitué, aryle, aryle substitué, hétéroaryle, hétéroaryle substitué, dans lequel R₇ est choisi parmi H, phényle, alkyle, et dans lequel R₈ et R₉ sont indépendamment sélectionnés dans le groupe constitué par H, alkyle, cycloalkyle, hétérocyclyle, hétérocyclyle substitué, aryle, aryle substitué, hétéroaryle, hétéroaryle substitué ou bien R₈ et R₉ sont liés entre eux pour former un cycle saturé de 5 à 8 chaînons contenant de 0 à 3 hétéroatomes choisis parmi O, S et N;
4) X est sélectionné dans le groupe constitué par: O et NH ;
5) R₃ est sélectionné dans le groupe constitué par : H, alkyle, alkyle substitué, cycloalkyle , cycloalkyle substitué ;
6) R4a est sélectionné dans le groupe constitué par : H et (C1-C4)alkyle ;
7) R4b est sélectionné dans le groupe constitué par : H et (C1-C4)alkyle ;
8) R₅ est sélectionné dans le groupe constitué par : H, halogène, R₁₀, CN, O(R₁₀), OC(O)(R₁₀), OC(O)N(R₁₀)(R₁₁), OS(O₂)(R₁₀), N(R₁₀)(R₁₁), N=C(R₁₀)(R₁₁), N(R₁₀)C(O)(R₁₁), N(R₁₀)C(O)O(R₁₁), N(R₁₂)C(O)N(R₁₀)(R₁₁), N(R₁₂)C(S)N(R₁₀)(R₁₁), N(R₁₀)S(O₂)(R₁₁), C(O)(R₁₀), C(O)O(R₁₀), C(O)N(R₁₀)(R₁₁), C(=N(R₁₁))(R₁₀), C(=N(OR₁₁))(R₁₀), S(R₁₀), S(O)(R₁₀), S(O₂)(R₁₀), S(O₂)O(R₁₀), S(O₂)N(R₁₀)(R₁₁) ; dans lequel chaque R₁₀, R₁₁, R₁₂ est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué,
le terme " substitué " faisant référence à un ou plusieurs substituants différents de H, choisi parmi halogène; alkyle; aryle; hétéroaryle, cycloalkyle ; hétérocyclyle ; alkylène; alkynyle; OH ; O-alkyle; O-alkylène ; O-aryle, O-hétéroaryle; NH₂ ; NH-alkyle; NH-aryle; NH-hétéroaryle; N-alkyle-alkyle' où alkyle' et alkyle sont deux alkyles identiques ou différents ; SH ; S-alkyle; S-aryle; S(O₂)H ; S(O₂)-alkyle; S(O₂)-aryle; SO₃H ; SO₃-alkyle; SO₃-aryle; CHO ; C(O)-alkyle; C(O)-aryle ; C(O)OH ; C(O)O-alkyle; C(O)O-aryle ; OC(O)- alkyle; OC(O)-aryle ; C(O)NH₂ ; C(O)NH-alkyle; C(O)NH-aryle ; NHCHO ; NHC(O)-alkyle; NHC(O)-aryle ; NH-cycloalkyle ; NH-hétérocyclyle.

Quand R₁ est H, les deux formes tautomères indiquées ci-dessous appartiennent à l'invention :

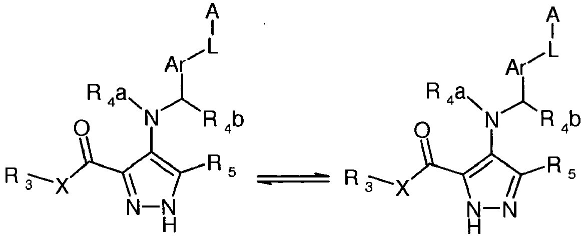

Dans les produits de formule (I), R₁ est avantageusement H.

Dans les produits de formule (I), R₃ est avantageusement H et X est avantageusement NH, ou bien, R₃ est avantageusement méthyle et X est avantageusement O.

Dans les produits de formule (I), R₅ est avantageusement H.

Un substituant Ar selon l'invention peut être choisi parmi phényle, pyridyle, thiényle, furyle, et pyrrolyle, substitué par R'₅, dans lequel R'₅ a la même définition que R₅.

Dans les produits de formule (I), Ar-L-A est avantageusement : 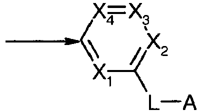 dans lequel chaque X₁, X₂, X₃ et X₄ est indépendamment choisi parmi N et C-R'₅, dans lequel R'₅ a la même définition que R₅.

Plus particulièrement, R'₅ peut être sélectionné dans le groupe constitué par H, F, Cl, méthyle, NH₂, OCF₃, et CONH₂. Un substituant Ar est avantageusement un phényle où R'₅ est H.

Un substituant A selon l'invention peut être sélectionné dans le groupe constitué par phényle, pyridyle, pyrimidyle, thiényle, furyle, pyrrolyle, oxazolyle, thiazolyle, isoxazolyle, isothiazolyle, pyrazolyle, imidazolyle, indolyle, indazolyle, benzimidazolyle, benzoxazolyle, et benzothiazolyle ; éventuellement substitué.

Plus particulièrement, un substituant A selon l'invention peut être sélectionné dans le groupe constitué par phényle, pyrazolyle et isoxazolyle ; éventuellement substitué. Un substituant A est avantageusement phényle, éventuellement substitué.

Parmi les produits de formule (I) objets de la présente invention, on peut notamment citer un premier groupe de produits pour lesquels R₄ₐ et R_{4b} sont H, et A, Ar, L, R₁, X, R₃ et R₅ sont tels que définis précédemment.

Parmi les produits du premier groupe on peut notamment citer un premier sous-groupe de produits pour lesquels :
1) A et Ar sont des phényles, éventuellement substitués;
2) L est sélectionné dans le groupe constitué par: NH-CO-NH et O-CO-NH;
3) X est NH et R₃ est H, ou bien X est O et R₃ est méthyle;
4) R₁, R₄ₐ, R_{4b} et R₅ sont H;

Parmi ce premier sous-groupe, on peut encore citer un sous-groupe de produits pour lesquels :
1) A et Ar sont des phényles, éventuellement substitués;
2) Lest NH-CO-NH;
3) X est NH et R₃ est H;
4) R₁, R₄ₐ, R_{ab} et R₅ sont H.

Parmi les produits du premier groupe on peut notamment citer un deuxième sous-groupe de produits pour lesquels :
1) A est un phényle éventuellement substitué et Ar est une pyridine, éventuellement substituée;
2) L est sélectionné dans le groupe constitué par: NH-CO-NH et O-CO-NH;
3) X est NH et R₃ est H, ou bien X est O et R₃ est méthyle;
4) R₁, R₄ₐ, R_{4b} et R₅ sont H;

Parmi ce deuxième sous-groupe, on peut notamment citer un sous-groupe de produits pour lesquels :
1) A est un phényle éventuellement substitué et Ar est une pyridine, éventuellement substituée;
2) Lest NH-CO-NH;
3) X est NH et R₃ est H;
4) R₁, R₄ₐ, R_{4b} et R₅ sont H;

Parmi les produits de formule (I) objets de la présente invention, on peut notamment citer un deuxième groupe de produits pour lesquels R₄ₐ est H, R_{4b} est (C1-C4)alkyle, et A, Ar, L, R₁, X, R₃ et R₅ sont tels que définis précédemment.

Parmi les produits du deuxième groupe on peut notamment citer un premier sous-groupe de produits pour lesquels :
1) A et Ar sont des phényles, éventuellement substitués;
2) L est NH-CO-NH;
3) X est NH et R₃ est H;
4) R₁, R₄ₐ et R₅ sont H;
5) R_{4b} est méthyle.

Parmi les produits de formule (I) objets de la présente invention, on peut notamment citer un troisième groupe de produits pour lesquels R₄ₐ est (C1-C4)alkyle, R_{4b} est H, et A, Ar, L, R₁, X, R₃ et R₅ sont tels que définis précédemment.

Parmi les produits du troisième groupe on peut notamment citer un premier sous-groupe de produits pour lesquels :
1) A et Ar sont des phényles, éventuellement substitués;
2) Lest NH-CO-NH;
3) X est NH et R₃ est H;
4) R₁, R_{4b} et R₅ sont H;
5) R₄ₐ est méthyle.

Parmi les produits du troisième groupe on peut notamment citer un deuxième sous-groupe de produits pour lesquels :
1) A et Ar sont des phényles, éventuellement substitués;
2) L est NH-CO-NH;
3) X est NH et R₃ est H;
4) R₁, R_{4b} et R₅ sont H;
5) R₄ₐ est éthyle.

A peut être substitué par un ou plusieurs substituants sélectionnés dans le groupe constitué par : H, F, Cl, Br, I, OH, SH, SO₃M, COOM, COO-alkyle, CON(R₁₄)(R₁₅), CN, NO₂, N(R₁₄)CO(R₁₅), N(R₁₄)(R₁₅), alkyle, alkyle halogéné, alkyle-OH, alkyle-N(R₁₄)(R₁₅), alkyle-(R₁₆), alkyle-COOM, alkyle-SO₃M, cycloalkyle, alkylène, alkynyle, aryle, hétéroaryle, O-alkyle, O-aryle, O-hétéroaryle, S-alkyle, S-aryle et S-hétéroaryle, chacun étant éventuellement substitué par un substituant choisi parmi alkyle, halogène, O-alkyle, N(R₁₄)(R₁₅) ; dans lequel R₁₄ et R₁₅ sont indépendamment choisis parmi H, alkyle, alkyle-OH, alkyle halogéné, alkyle-NH₂, alkyle-COOM, alkyle-SO₃M ; dans lequel lorsque R₁₄ et R₁₅ sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R₁₆ est H ou un hétérocycle non aromatique éventuellement substitué, comprenant 2 à 7 atomes de carbone, et 1 à 3 hétéroatomes choisis parmi N, O et S. Lorsque A est disubstitué, les deux substituants peuvent être liés entre eux pour former un cycle de 5 à 7 chaînons contenant de 0 à 3 hétéroatomes choisis parmi N, O et S.

A peut encore être substitué par un ou plusieurs substituants sélectionnés dans le groupe cité ci-dessus complété par SiMe₃, S-CHF₃, SF₅.

Plus particulièrement, A peut être choisi parmi phényle, pyrazolyle ou isoxazolyle, substitué par au moins un groupe choisi parmi H, halogène, alkyle, alkyle halogéné, O-alkyle, COO-alkyle, O-alkyle halogéné. Un substituant A est avantageusement sélectionné dans le groupe constitué par : phényl, 2-fluoro-5-(trifluorométhyl)phényl, 2-fluorophényl, 2-méthoxyphényl, 2-fluoro-3-(trifluorométhyl)phényl, 3-méthoxyphényl, 3-fluoro-5-(trifluorométhyl)phényl, 4-(trifluorométhoxy)phényl, 3-méthoxycarbonylphényl, 4-(trifluorométhyl)phényl, 3-(trifluorométhyl)phényl, 2-(trifluorométhyl)phényl, 3,5-diméthoxyphényl, 3-méthylphényl, 4-méthoxyphényl, 4-fluorophényl, 4-chloro-3-(trifluorométhyl)phényl, 4-(difluorométhoxy)phényl, 2-chloro-4-(trifluorométhyl)phényl, 4-méthylphényl, 2,5-diméthylphényl, 3,4-diméthylphényl, 2-méthylphényl, 3-éthylphényl, 3,5-bis(trifluorométhyl)phényl, 3-fluorophényl, 2-méthoxy-5-méthylphényl, 2,5-diméthoxyphényl, 3-chloro-4-(difluorométhoxy)phényl, 2,5-difluorophényl et 4-méthyl-3-(trifluorométhyl)phényl.

Un substituant A est encore avantageusement sélectionné dans le groupe constitué par : 2-chloro-5-trifluorométhyl-phényl, 3-chloro-4-fluoro-phényl, 3,4-dichloro-phényl, 3-chloro-5-trifluorométhyl-phényl, 3-triméthylsilyl-4-fluoro-phényl, 3-trifluorométhoxy-phényl, 4-trifluorométhyl-pyridin-2-yl, 4-méthoxy-pyridin-2-yl, 3-trifluorométhyl-4-chloro-phényl, 2-chloro-5-trifluorométhyl-phényl, 3-trifluorométhylsulfanyl-phényl, 3-isopropyl-phényl, 3-isopropyl-4-fluoro-phényl, 3-pentafluorosulfanyl-phényl, 2-méthoxy-5-tertiobutyl-phényl, 4-isopropyl-phényl, 2-chloro-4-isopropyl-phényl, 2-fluoro-5-méthyl-phényl, 2-fluoro-4-trifluorométhyl-phényl, 2-fluoro-4-méthyl-phényl, 2-chloro-4-méthyl-phényl et 2-chloro-5-méthyl-phényl.

La présente invention comprend aussi les objets correspondant aux combinaisons des sous-groupes cités ci-dessus.

Les produits selon l'invention peuvent être sous forme :
1) non chirale, ou
2) racémique, ou
3) enrichie en un stéréo-isomère, ou
4) enrichie en un énantiomère ;
et être éventuellement salifiés.

La présente invention concerne aussi les compositions pharmaceutiques comprenant un produit selon l'invention, en combinaison avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et de ce fait auront une formulation acceptable pour une telle utilisation.

Des voies d'administration par injection acceptables incluent les voies intraveineuse, intrapéritonéale, intramusculaire, et sous cutanée, la voie intraveineuse étant habituellement préférée.

La dose administrée des composés de l'invention sera adaptée par le praticien en fonction de la voie d'administration au patient et de l'état de ce dernier.

Il est décrit des composés utiles comme agents inhibiteurs d'une ou plusieurs réactions catalysées par une kinase. KDR et/ou Tie2 sont des kinases pour lesquelles des composés peuvent être utiles en tant qu'inhibiteurs.

Les raisons pour lesquelles ces kinases sont choisies sont données ci-après :

### KDR

KDR (Kinase insert Domain Receptor) aussi appelée VEGF-R2 (Vascular Endothelial Growth Factor Receptor 2), est principalement exprimé dans les cellules endothéliales. Ce récepteur lie le facteur pro-angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

### Tie2

Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïétine 1 ou Ang1) qui stimule l'autophosphorylation du récepteur et la signalisation cellulaire [S. Davis et al (1996) Cell 87, 1161-1169] et l'antagoniste (angiopoïétine 2 ou Ang2) [P.C. Maisonpierre et al. (1997) Science 277, 55-60]. L'angiopoïétine 1 peut avoir un effet synergique avec le VEGF dans les derniers stades de la néo-angiogénèse [AsaharaT. Circ. Res.(1998) 233-240]*.* Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180*].* La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60*].* Lin et al (1997) J. Clin. Invest. 100, 8: 2072-2078 *et* Lin P. (1998) PNAS 95, 8829-8834*,* ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénogreffes de tumeur du sein et de mélanome.

Pour les raisons qui suivent, les inhibiteurs de Tie2 peuvent être utilisés dans les situations où une néovascularisation ou angiogenèse se fait de façon inappropriée c'est-à-dire dans les cancers en général mais aussi dans des cancers particuliers tels que le sarcome de Kaposi ou l'hémoangiome infantile, l'arthrite rhumatoïde, l'osthéoarthrite et/ou ses douleurs associées, les maladies inflammatoires de l'intestin telle que la recto-colite hémorragique ou la maladie de Crohn's, les pathologies de l'oeil telle que la dégénérescence maculaire liée à l'âge, les rétinopathies diabétiques, l'inflammation chronique, le psoriasis.

L'angiogenèse est un processus de génération de nouveaux vaisseaux capillaires à partir des vaisseaux pré-existants. L'angiogenèse tumorale (formation de néovaisseaux sanguins), indispensable à la croissance tumorale, est également un des facteurs essentiels de la dissémination métastatique (Oncogene. 2003 May 19;22(20):3172-9 ; Nat Med. 1995 Jan;1(1):27-31.).

Cette néo-vascularisation est due à la migration, puis à la prolifération et à la différenciation des cellules endothéliales sous l'influence de facteurs angiogéniques sécrétés par les cellules cancéreuses et les cellules du stroma (Recent Prog Horm Res. 2000;55:15-35; 35-6).

Le système angiopoïétine 1 / récepteur Tie2 joue un rôle prépondérant dans la maturation des vaisseaux en permettant le recrutement de cellules péri-endothéliales pour stabiliser le vaisseau (Cell. 1996 Dec 27;87(7):1161-9, Recent Prog Horm Res. 2004;59:51-71). Ainsi il a été montré que l'administration de la forme recombinante soluble du domaine extracellulaire du récepteur Tie-2 (exTek) inhibe l'angiogenèse tumorale dans des modèles de tumeurs murines ainsi que le développement métastatique (Proc Natl Acad Sci USA. 1998 Jul 21;95(15):8829-34 ; Cancer immunol Immunother. 2004 Jul;53(7):600-8). Dans des cellules endothéliales en culture, la stimulation de Tie-2 active la voie de la PI3 kinase, de p42/p44 voies impliquées dans la prolifération et la migration cellulaire; de la synthèse de PAF (Cell Signal. 2006 Apr 14; ahead of print) voie impliquée dans l'activité pro-inflammatoire. La stimulation de Tie2 stimule la voie de l'Akt et inhibe l'apoptose (Exp Cell Res. 2004 Aug 1;298(1):167-77) une voie de transduction connue pour son importance dans la survie cellulaire.

L'ajout de Extek (récepteur soluble de Tie2) inhibe la formation de pseudo tubules des cellules endothéliales sur Matrigel (Cancer immunol Immunother. 2004 Jul; 53(7): 600-8. Ces études indiquent que le système Tie-2/Angiopoiétine est nécessaire lors des premiers stades de la formation de bourgeons vasculaires dans les tissus adultes et qu'une fonction du récepteur Tie-2 est d'augmenter la survie des cellules endothéliales au cours de la formation des vaisseaux sanguins. De plus, l'angiopoietine-1 stimule la prolifération des cellules endothéliales lymphatiques ainsi que la lymphangiogenèse (développement des néo-vaisseaux lymphatiques) voie d'accès privilégiés pour le développement métastatique (Blood. 2005 Jun 15; 105(12): 4649-56)

Les processus d'angiogenèse jouent ainsi un rôle prépondérant dans la progression de nombreuses tumeurs solides. De plus, il a été montré que la probabilité d'apparition de métastases augmente très fortement avec l'augmentation de la vascularisation de la tumeur primaire (Br J Cancer. 2002 May 20 ; 86(10): 1566-77.

Le rôle potentiel d'agents pro-angiogéniques dans les leucémies et lymphomes a été également et plus récemment documenté. En effet de manière générale il a été rapporté que des clones cellulaires dans ces pathologies peuvent être soit détruits naturellement par le système immunitaire soit basculer dans un phénotype angiogénique qui favorise leur survie puis leur prolifération. Ce changement de phénotype est induit par une sur expression de facteurs angiogéniques notamment par les macrophages et/ou une mobilisation de ces facteurs à partir de la matrice extracellulaire (Thomas DA, Giles FJ, Cortes J, Albitar M, Kantarjian HM. , Acta Haematol, (2001), vol 207, pp106-190.

Il existe une corrélation entre le processus d'angiogenèse de la moelle osseuse et les "extramedullar disease" dans les CML (chronic myelomonocytic leukemia). Différentes études démontrent que l'inhibition de l'angiogenèse, pourrait représenter une thérapeutique de choix dans cette pathologie (Leuk Res. 2006 Jan; 30(1): 54-9 ; Histol Histopathol. 2004 oct. ;19(4): 1245-60. De plus, il est fortement suggéré que l'activation du système Tie2/angiopoietine soit impliquée dans le développement de l'angiogenèse de la moelle osseuse chez les patients atteints de myélome multiple (Blood. 2003 Jul 15; 102(2): 638-45.

L'arthrite rhumatoïde (RA) est une maladie chronique avec une étiologie inconnue. Alors qu'elle affecte de nombreux organes, la forme la plus sévère de RA est une inflammation synoviale des articulations progressive aboutissant à la destruction. L'angiogenèse semble affecter de manière importante la progression de cette pathologie. Ainsi, il a été montré que l'activation de Tie2 régule l'angiogenèse dans les tissus synoviaux favorisant le développement de l'arthrite rhumatoïde (Arthritis Rheum. 2003 Sep; 48(9): 2461-71)

Il a été montré également une sur expression de L'angiopoientin 1 et de Tie2 dans les tissus synoviaux de patients atteints d'ostheoarthrite corrélée à une néovascularisation active (Shahrara S et al Arthritis Res. 2002;4(3)). Ainsi, il a été montré qu'en bloquant l'activation de Tie2 par l'utilisation d'un adénovirus produisant exTEK (récepteur Tie2 soluble) on obtient une inhibition de l'angiogenèse, du développement de l'arthrose et une protection de la dégradation osseuse dans un modèle de souris où l'arthrose est induite au collagène (Arthritis Rheum. 2005 May; 52(5):1346-8).

Les IBD (inflammatory bowel disease) comprennent deux formes de maladies inflammatoires chroniques de l'intestin : les UC (ulcerative colitis) et la maladie de Crohn's (CD). Les IBD sont caractérisées par une dysfonction immunitaire se traduisant par une production inappropriée de cytokines inflammatoires induisant l'établissement d'un système micro-vasculaire local. Cette angiogenèse d'origine inflammatoire a pour conséquence une ischémie intestinale induite par vasoconstriction (Inflamm Bowel Dis. 2006 Jun;12(6):515-23).

Les pathologies de l'oeil en relation avec des phénomènes de néo vascularisation comme la dégénérescence maculaire liée à l'age sont responsables d'une large majorité des cas de cécité dans les pays développés. Les signaux moléculaires qui contrôlent les phénomènes de néo vascularisation dans l'oeil tels que les VEGFs ou les angiopoiétines sont des cibles de choix dans ces pathologies (Campochiaro PA. Expert Opin Biol Ther. 2004 Sep;4(9)). Ainsi, il a été montré qu'en bloquant l'activation de Tie2 par l'utilisation d'un adénovirus produisant exTEK (récepteur Tie2 soluble) inhibait la néovascularisation rétinienne et choroïdienne qui est la cause la plus fréquente de la perte de vision (Hum Gene Ther. 2001 Jul 1; 12(10):1311-21)

Un produit conforme à l'invention pourra être utilisé pour la fabrication d'un médicament utile pour traiter un état pathologique, en particulier un cancer.

Grâce à leur faible toxicité et leurs propriétés pharmacologiques et biologiques, les composés de la présente invention trouvent leur application dans le traitement de tout carcinome ayant un degré de vascularisation important ou induisant des métastases ou enfin dans des pathologies de type lymphomes et leucémies.

Ces composés représentent une thérapie de choix soit seul soit en association avec une chimiothérapie ou radiothérapie adaptée et/ou en association avec d'autres composés possédant des activités anti-angiogènes comme les inhibiteurs des VEGFs ou des FGFs. Ainsi les produits de formule générale (I) sont notamment utiles pour le traitement ou la prévention d'un état pathologique caractérisé en ce que le produit est administré seul ou en association avec d'autres principes actifs notamment des anti-cancéreux tels que des produits cytotoxiques, cytostatiques, anti-angiogéniques, ou anti-métastasiques.

Les composés de la présente invention peuvent donc être administrés seuls ou en mélange avec d'autres anticancéreux. Parmi les associations possibles on peut citer:
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoïdes (paclitaxel et docétaxel)
- les anthracyclines comme notamment la doxorubicine, la daunorubicine, l'idarubicine, l'épirubicine, la mitoxantrone, la losoxantrone
- les inhibiteurs de topoisomérases des groupes I et II telles que l'étoposide, le teniposide, l'amsacrine, l'irinotecan, le topotecan et le tomudex
- les fluoropyrimidines telles que la 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine tels que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptine ainsi que les hormones oestrogéniques, androgéniques
- les agents antivasculaires tels que les dérivés de la combretastatine, par exemple la CA4P, des chalcones ou de la colchicine, par exemple le ZD6126, et leurs prodrogues
- les agents anti-angiogéniques tels que le bevacizumab, le sorafenib ou le sunitinib malate
- les agents thérapeutiques inhibiteurs d'autres tyrosine kinases tels que l'imatinib, le gefitinib et l'erlotinib.

Lorsque les composés de la présente invention sont associés à un autre traitement ou à un traitement par des radiations, ces traitements peuvent alors être administrés simultanément, séparément, séquentiellement. Le traitement sera adapté par le praticien en fonction du malade à traiter.

### Définitions

Le terme « halogène » fait référence à un élément choisi parmi F, Cl, Br, et I.

Le terme « alkyle » fait référence à un substituant hydrocarboné saturé, linéaire ou ramifié, ayant de 1 à 6 atomes de carbone. Les substituants méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthyl-propyle, 1-éthylpropyle, hexyle, 1-méthylpentyle, 2-méthylpentyle, 1-éthylbutyle, 2-éthylbutyle, 3,3-diméthylbutyle, sont des exemples de substituant alkyle.

Le terme « alkylène » fait référence à un substituant hydrocarboné linéaire ou ramifié ayant une ou plusieurs insaturations, ayant de 2 à 6 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 6atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tétrahydronapht-5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle ; oxadiazolyle ; thiadiazolyle ; tétrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle ; cyclooctyle; bicyclo[2.2.2]octyle ; adamantyle ; et perhydronapthyle sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Le terme « substitué » fait référence à un ou plusieurs substituants différents de H, par exemple halogène; alkyle; aryle; hétéroaryle, cycloalkyle ; hétérocyclyle ; alkylène; alkynyle; OH ; O-alkyle; O-alkylène ; O-aryle; O-hétéroaryle; NH₂ ; NH-alkyle; NH-aryle; NH-hétéroaryle; N-alkyle-alkyle' où alkyle' et alkyle sont deux alkyles identiques ou différents ; SH ; S-alkyle; S-aryle; S(O₂)H ; S(O₂)-alkyle; S(O₂)-aryle; SO₃H ; SO₃-alkyle; SO₃-aryle; CHO ; C(O)-alkyle; C(O)-aryle ; C(O)OH ; C(O)O-alkyle; C(O)O-aryle ; OC(O)-alkyle; OC(O)-aryle ; C(O)NH₂ ; C(O)NH-alkyle; C(O)NH-aryle ; NHCHO ; NHC(O)-alkyle; NHC(O)-aryle ; NH-cycloalkyle ; NH-hétérocyclyle.

La présente invention a encore pour objet le procédé de préparation des produits de formule générale (I) suivante : 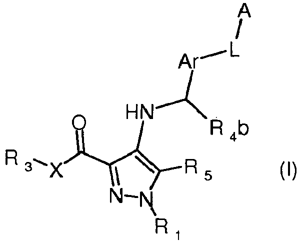 dans laquelle R₁, R₃, R_{4b}, R5, X, Ar, L et A sont tels que définis précédemment caractérisé en ce qu'un produit de formule générale (II) suivante : 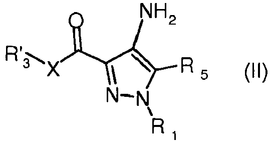 dans laquelle R'₃ est R₃ ou un précurseur de R₃, et X, R₁, R₃ et R₅ sont tels que définis précédemment, réagit avec un produit de formule (III) suivante : 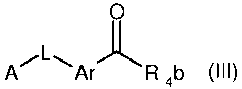 dans laquelle R_{4b}, Ar, L et A sont tels que définis précédemment, pour obtenir le produit de formule générale (I).

Il est décrit le procédé de préparation de produits intermédiaires de formule générale (II) suivante : 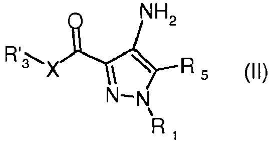 dans laquelle R'₃, X, R₁ et R₅ sont tels que définis précédemment, caractérisé en ce qu'un produit de formule générale (IV) suivante : 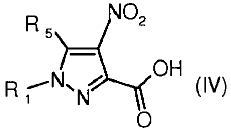 dans laquelle R₁ et R₅ sont tels que définis précédemment, réagit avec un produit de formule générale (V) suivante : 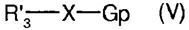 dans laquelle Gp est un groupe protecteur, X et R'₃ sont tels que définis précédemment, pour obtenir le produit de formule générale (II).

Il est également décrit le procédé de préparation des produits intermédiaires de formule générale (III) suivante : 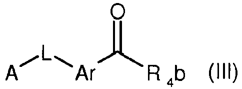 dans laquelle R_{4b}, Ar et A sont tels que définis précédemment, et L est NH-CO-NH, caractérisé en ce qu'un produit de formule générale (VI) suivante : 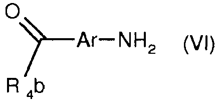 dans laquelle R_{4b}, Ar est tel que défini précédemment, réagit avec un produit de formule générale (VII) suivante : 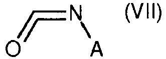 dans laquelle A est tel que défini précédemment, pour obtenir le produit de formule générale (III).

La présente invention a encore pour objet un procédé de préparation des produits de formule générale (I) 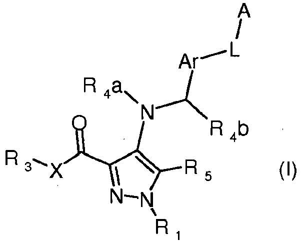 dans laquelle R₁, R₃, R₄ₐ, R_{4b},R5, X, Ar et A sont tels que définis précédemment, et L est NHCONH, caractérisé en ce qu'un produit de formule générale (VIII) suivante : 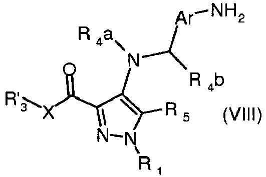 dans laquelle R'₃ est R₃ ou un précurseur de R₃, et X, R₁, R₃, R₄ₐ, R_{4b} et R₅ sont tels que définis précédemment, réagit avec un produit de formule (VII) suivante : 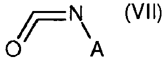 dans laquelle A est tel que défini précédemment, pour obtenir le produit de formule générale (I') de formule suivante 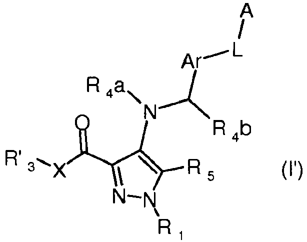 où le précurseur R'₃ est transformé en R₃ afin d'obtenir le produit de formule générale (I).

On décrit également le procédé de préparation des produits intermédiaires de formule générale (VIII) 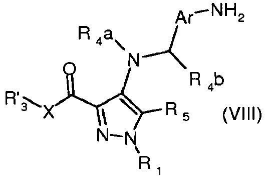 dans laquelle R'₃ est R₃ ou un précurseur de R₃, et Ar, X, R₁, R₃, R₄ₐ, R_{4b} et R₅ sont tels que définis précédemment, caractérisé en ce que un produit de formule générale (IX) suivante : 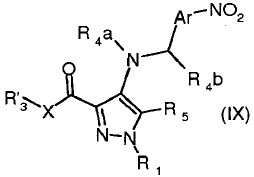 subit une réduction pour obtenir un produit de formule générale (VIII).

On décrit également le procédé de préparation des produits intermédiaires de formule générale (IXa) 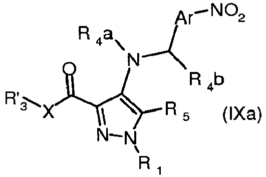 dans laquelle R'₃ est R₃ ou un précurseur de R₃, R₄ₐ est (C1-C4)alkyle, et R_{4b} est H, et X, R₁, R₃ et R₅ sont tels que définis précédemment, caractérisé en ce que un produit de formule générale (IXc) suivante : 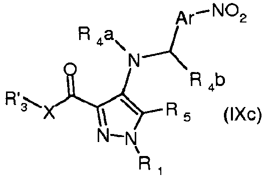 dans laquelle R'₃ est R₃ ou un précurseur de R₃, R₄ₐ et R_{4b} sont H, et X, R₁, R₃ et R₅ sont tels que définis précédemment, subit une aminoalkylation pour obtenir un produit de formule générale (IXa) ci-dessus.

On décrit également le procédé de préparation des produits intermédiaires de formule générale (IXc) 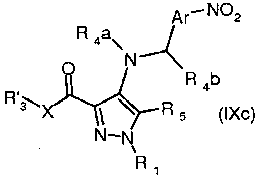 dans laquelle R'₃ est R₃ ou un précurseur de R₃, R₄ₐ et R_{4b} sont H, et Ar, X, R₁, R₃ et R₅ sont tels que définis précédemment, caractérisé en ce que un produit de formule générale (II) suivante : 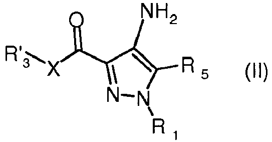 dans laquelle R'₃, X, R₁ et R₅ sont tels que définis précédemment, réagit avec un produit de formule générale (Xc) suivante 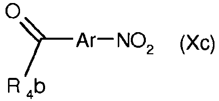 dans laquelle R_{4b} est H et Ar est tel que défini précédemment pour obtenir un produit de formule générale (IXc) ci-dessus.

On décrit également le procédé de préparation des produits intermédiaires de formule générale (IXb) 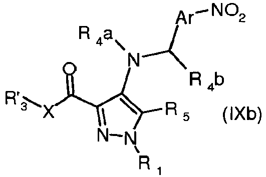 dans laquelle R'₃ est R₃ ou un précurseur de R₃, R₄ₐ est H, et R_{4b} est (C1-C4)alkyle, et Ar, X, R₁, R₃ et R₅ sont tels que définis précédemment, caractérisé en ce que un produit de formule générale (II) suivante : 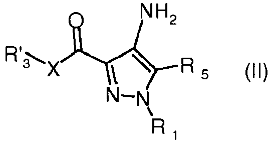 dans laquelle R'₃, X, R₁ et R₅ sont tels que définis précédemment, réagit avec un produit de formule générale (Xb) suivante 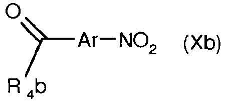 dans laquelle R_{4b} est (C1-C4)alkyle et Ar est tel que défini précédemment puis subit une réduction pour obtenir un produit de formule générale (IXb) ci-dessus.

Les produits de départs utilisés sont disponibles commercialement ou sont préparés par des méthodes connues de l'homme de l'art.

On entend par groupe protecteur Gp un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont donnés dans « Protective Groups in Organic Synthesis », Green et al., 2nd Edition (John Wiley & Sons, Inc., New York).

On entend par précurseur de R₃ est un groupe qui permet de générer, en fin de réaction ou de synthèse, un groupe R₃. Il s'agit, par exemple, d'un groupe -CH₂-résine Rink lorsque X est NH, ou encore d'un groupe 2,4-diméthoxybenzyl.

Les produits selon l'invention peuvent être préparés à partir de méthodes conventionnelles de chimie organique. Le schéma 1 ci-dessous est illustratif de la méthode utilisée pour la préparation des exemples 1 à 31. A ce titre, elle ne saurait constituer une limitation de la portée de l'invention, en ce qui concerne les méthodes de préparation des composés revendiqués.

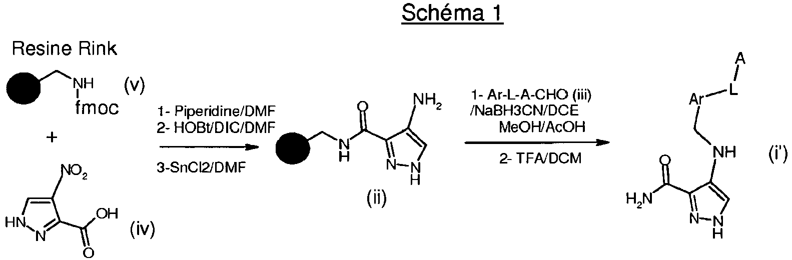

### 1- Préparation de la résine intermédiaire (ii)

La résine Rink (v) est gonflée dans le DMF. Le DMF est filtré puis remplacé par une solution à 50% de pipéridine dans le DMF. Après ½ heure d'agitation à température ambiante le mélange est filtré puis la résine est successivement lavée au DMF, méthanol et DMF.

Ensuite une solution de 3 équivalents d'acide 4-nitro-3-pyrazole carboxylique (iv), 3 équivalents de HOBt (hydroxybenzotriazole) et 3 équivalents de Diisopropyle carbodiimide (DIC) dans le DMF est additionnée à la résine. Le mélange est agité une nuit à température ambiante puis le mélange réactionnel est éliminé par filtration. La résine est lavée 3 fois au DMF, 2 fois au méthanol et 5 fois au DMF. La résine est ensuite traitée avec une solution molaire de chlorure d'étain (SnCl₂), une nuit à température ambiante. Le mélange réactionnel est éliminé par filtration. La résine est lavée 5 fois au DMF, 2 fois au méthanol et 3 fois au DCM (dichlorométhane) et 2 fois a l'éther puis séchée sous vide pour donner la résine (ii).

### 2- Préparation des produits (i') par amination réductive

Dans un vial, la résine (ii) est gonflée dans le dichloroéthane (DCE). 3 équivalents d'aldéhyde (iii) en solution dans le DMF sont additionnés suivie de 5 équivalents de cyanoborohydrure de sodium dans le méthanol contenant 10% d'acide acétique. Le mélange est porté à 80°C pendant 2 heures. Après refroidissement, le milieu réactionnel est filtré et la résine lavée successivement 2 fois avec du méthanol, 3 fois avec du DCM (dichlorométhane), 2 fois avec du méthanol et 3 fois avec du DCM. Les produits finaux sont obtenus par clivage avec une solution d'acide trifluoroacétique/DCM (50/50) à température ambiante pendant 2 heures. Le produit est isolé par filtration et évaporation du solvant. Le produit (i') est purifié soit par chromatographie liquide en phase normale ou par LC/MS préparative.

### Matériel et méthodes

### LC/MS méthode analytique A:

L'analyse est effectuée sur un spectromètre de masse Waters, modèle ZQ en mode electrospray négatif et positif (gamme de 10 a 1200 amu) connecté a un instrument HPLC Agilent HP 1100. La séparation est effectuée sur une colonne Waters Xbridge C18 (3X50 mm, 2.5 µm diamètre des particules) maintenue a 60°C, en utilisant un gradient acétonitrile/eau contenant 0.1% (v/v) d'acide formique et a un débit de 1.1 ml/min. Le gradient passe de 5 à 100% d'acétonitrile en 5 minutes, maintenu à 100% ½ minute puis ramené a 5% en 1 minute. La durée totale de la séparation est de 7 minutes. En plus de l'analyse de spectroscopie de masse, une détection UV (diode array) est effectuées aux longueurs d'onde de 210 à 400 nM ainsi qu'une mesure ELSD (evaporative light scattering) à l'aide d'un instrument Sedere Sedex 85.

### LC/MS méthode analytique B:

L'analyse est effectuée sur un spectromètre de masse Waters, modèle ZQ en mode electrospray négatif et positif (gamme de 10 a 1200 amu) connecté a un instrument Waters Alliance HT. La séparation est effectuée sur une colonne Waters Atlantis dC18 column (2.1x50 mm, 5 µm diamètre des particules) maintenue a 25°C, en utilisant un gradient acétonitrile/eau contenant 0.1% (v/v) d'acide trifluoroacétique et à un débit de 0.5 ml/min. Le gradient passe de 5 à 85% d'acétonitrile en 5 minutes, puis maintenu à 90% pendant 1 minute. La durée totale de la séparation est de 7 minutes. En plus de l'analyse de spectroscopie de masse, une détection UV (diode array) est effectuée aux longueurs d'onde de 210 à 400 nM.

### LC/MS méthode préparative C:

Les produits sont purifiés par LC/MS préparative en utilisant un système Waters FractionLynx composé d'une pompe Waters modèle 600 pour le gradient, une pompe Waters modèle 515 pour la régénération, d'une pompe Waters Reagent Manager, d'un auto-injecteur modèle 2700, 2 interrupteurs Theodyne modèle LabPro, un détecteur Waters à diode array, un spectromètre de masse Waters modèle ZMD et un collecteur de fractions modèle 204. L'instrument est piloté par le logiciel Waters FractionLynx. En sortie de colonne de séparation, 1/1000 du flot est dérivé grâce à un splitter LC Pcking Accurate; ce flot est mélangé à du méthanol (débit, 0.5 ml/min) et envoyé vers les détecteurs: % est envoyé vers la diode array et ¼ vers le spectromètre de masse; le reste du flot (999/1000) est envoyé vers le collecteur de fractions. Le produit est collecté si le pic de masse est observé par FractionLynx, sinon le flot est directement éliminé. Les formules moléculaires des produits sont transmises au logiciel fractionLynx et le produit est collecté lorsque les pics de masse [M+H]⁺ et [M+Na]⁺ sont détectés. Les fractions sont collectées dans des vials qui sont évaporés dans un évaporateur rotatif Jouan modèle RC10.10. Le poids de produit obtenu est déterminé en mesurant le poids du vial après évaporation du solvant. Les informations sur les colonnes et les gradients utilisés sont donnés pour chaque exemple dans la partie suivante.

### LC/MS méthode analytique D:

L'analyse est effectuée sur un spectromètre de masse Waters SQD en mode electrospray négatif et positif (gamme de 10 a 1200 amu) connecté a un instrument Waters Alliance HT. La séparation est effectuée sur une colonne BEH (2.1x50 mm, 1.7 µm diamètre des particules) maintenue à 25°C, en utilisant un gradient acétonitrile/eau contenant 0.1% (v/v) d'acide trifluoroacétique et à un débit de 1 ml/min. Le gradient passe de 5 à 100% d'acétonitrile en 2 minutes. En plus de l'analyse de spectroscopie de masse, une détection UV (diode array) est effectuée aux longueurs d'onde de 210 à 400 nM

### Exemple 1: Trifluoroacétate de 4-{[3-phényl]carbamoyl}oxy)benzyl] amino}-1H-pyrazole-3-carboxamide

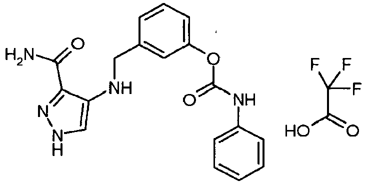

### Préparation de la résine (ii) :

30 g de résine Rink (PolymerLab ; 0.99 mmol/g) est gonflée dans 150 ml de DMF. Après 10 min d'agitation, le DMF est filtré et remplacé par 150 ml d'une solution de pipéridine dans le DMF (50/50, v/v). Le mélange est maintenu 1 heure sous agitation puis filtré. La résine est lavée successivement avec 5 fois 150 ml de DMF, 2 fois 150 ml de méthanol et 3 fois 150 ml de DMF. Ensuite une solution de 14.1 g d'acide 4- nitro-3-pyrazole carboxylique (90 mmoles, 3 équi.) et 13.8 g de HOBt (90 mmoles, 3équi.) dans 150 ml de DMF est additionnée à la résine immédiatement suivie de 13.8 ml de DIC (90 mmoles ; 3 équi.). Le mélange est maintenu 16 heures sous agitation a température ambiante. La solution est filtrée, la résine est lavée successivement avec 5 fois 150 ml de DMF, 2 fois 150 ml de méthanol et 3 fois 150 ml de DMF puis 150 ml d'une solution 1 M de SnCl2 (33 g dans 150 ml). L'agitation a température ambiante est maintenue 24 heures puis le mélange est filtré et la résine lavée avec 5 fois 150 ml de DMF, 2 fois 150 ml de méthanol, 3 fois 150 ml de DCM et 2 fois 150 ml d'éther éthylique. Après séchage sous vide on isole 31 g de résine intermédiaire (ii).

### Préparation de l'exemple 1 :

100 mg de résine (ii) est gonflée dans 0.3 ml de DCE, puis 72 mg d'acide phényl-carbamic -3-formyl-phényl ester (0.3 mmoles ; ∼ 3 équi.) en solution dans 0.2 ml de DMF sont additionnés suivis de 33 mg de cyanoborohydrure de sodium (0.5 mmoles ; ∼5 équi.). Le mélange est chauffé à 80°C pendant 1 heure, puis filtré après refroidissement à température ambiante. La résine est ensuite lavée successivement avec 2 fois 1 ml de MeOH, 5 fois 1 ml de DMF, 3 fois 1 ml de MeOH et 5 fois 1 ml de DCM. Le produit est clivé par traitement de la résine avec 1 ml d'une solution de TFA/DCM à 50/50. La solution est évaporée et le brut obtenu est directement purifié par HPLC préparative. 2.4 mg de produit attendu 1 est obtenu (rdt=5%). ([M+H]+): 352). RT= 2.49 min (Méthode A)

### Exemple 2a : Trifluoroacétate de 4-{[3-({[2-fluoro-5-(trifluorométhyl) phényl]carbamoyl}amino)benzyl]amino}-1H-pyrazole-3-carboxamide et Exemple 2b : Chlorhydrate de 4-{[3-({[2-fluoro-5-(trifluorométhyl) phényl]carbamoyl}amino)benzyl]amino}-1H-pyrazole-3-carboxamide

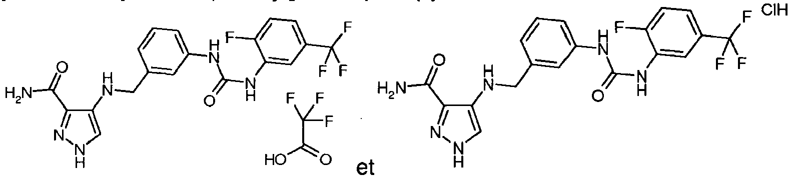

### Préparation de la 1-(2-Fluoro-5-trifluorométhyl-phényl)-3-(3-formyl-phényl)-urée :

Un mélange de 484 mg 3-aminobenzaldéhyde (4 mmoles) (forme polymérisée) et 290 µl de 2-fluoro-5-trifluorométhyl-phényl isocyanate dans 4 ml de DCE est traité dans un four à micro-onde CEM Discover à 100°C pendant 10 minutes (puissance 90). Après refroidissement, le mélange est versé dans 100 ml d'une solution saturée d' hydrogénosulfate de potassium et extrait par deux fois 50 ml d'acétate d'éthyle. Les phases organiques combinées sont lavées à l'eau, séchées sur sulfate de sodium et évaporée. On isole 740 mg d'aldéhyde attendu (rdt= 57%) avec une pureté LC/MS de 82%. Le produit brut est directement utilisé pour les étapes suivantes. ([M+H]+): 327. RT= 4.28 min (gradient acétonitrile/eau de 30 a 90%- méthode B).

### Préparation de l'exemple 2a :

L'exemple 2a a été préparé selon la méthode décrite pour l'exemple 1 en partant de 1 g de résine (ii), 520 mg de 1-(2-Fluoro-5-trifluorométhyl-phényl)-3-(3-formyl-phényl)- urée (1.6 mmole, 2 équi.) et 264 mg de cyanoborohydrure de sodium (4 mmoles ; ∼5 équi.). Après purification par HPLC préparative, on obtient le produit 2a. (EIMS ([M+H]+): 437. RT= 3.45min.(méthode A).

### Préparation de l'exemple 2b :

L'exemple 2b a été préparé selon la méthode décrite pour l'exemple 1 en partant de 1g de résine (ii), 520 mg de 1-(2-Fluoro-5-trifluorométhyl-phényl)-3-(3-formyl-phényl)- urée (1.6 mmole, 2 équi.) et 264 mg de cyanoborohydrure de sodium (4 mmoles ; ∼5 équi.). Après filtration et évaporation on isole 301 mg de produit brut (pureté LC/MS 83%). Le produit brut est purifié sur colonne de silice en utilisant un mélange DCM/MeOH (90/10) comme éluant). 151 mg de solide jaune pale sont isolés (rdt 43%). Le produit est dissous dans 2 ml de MeOH et est transformé en son sel de chlorhydrate par addition d'une solution 4N de HCl dans le dioxanne. Après évaporation, on isole le produit 2b sous forme de solide jaune pale. (EIMS ([M+H]+): 437. RT= 3.45min.(méthode A). H¹NMR (D₆-MeOD) (600MHz): 4.62 (s, 2H); 7.19 (d, 1 H); 7.36 (2s, 2H); 7.40 (m, 1 H); 7.49 (m, 1 H); 7.76 (s, 1 H); 7.78 (s, 1 H); 8.62 (m, 1 H)

### Exemple 3 : Trifluoroacétate de 4-[(3-{[(2-fluorophényl) carbamoyl]amino}benzyl)amino]-1H-pyrazole-3-carboxamide

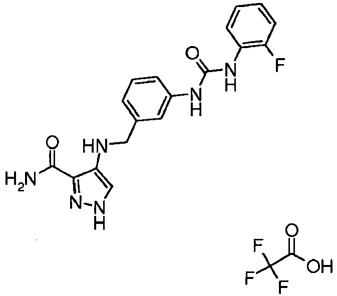

### Préparation de la 1-(2-Fluoro-phényl)-3-(3-formyl-phényl)-urée :

Le produit a été préparé tel que décrit dans l'exemple 2 à partir de 484 mg de 3-aminobenzaldéhyde (4 mmoles) (forme polymérisée) et 548 mg de 2-fluoro-phényl isocyanate dans 4 ml de DCE. Après refroidissement, le mélange est versé dans 100ml d'une solution saturée de potassium hydrogénosulfate et extrait par deux fois 50 ml d'acétate d'éthyle. Les phases organiques combinées sont lavées à l'eau, séchées sur sulfate de sodium et évaporée. On isole 864 mg d'aldéhyde attendu sous forme de gomme avec une pureté LC/MS de 77%. Une fraction cristallisée est obtenue après trituration dans l'éther éthylique. On isole 165 mg de solide ([M+H]+): 259. RT= 4.28 min (gradient acétonitrile/eau de 5 à 85%- méthode B).

### Préparation de l'exemple 3:

L'exemple 3 a été préparé selon la méthode décrite pour l'exemple 1 en partant de 200 mg de résine (ii), 104 mg de 1-(2-Fluoro-phényl)-3-(3-formyl-phényl)-urée (0.4 mmole, 2 équi.) et 66 mg de cyanoborohydrure de sodium (1 mmole ; ∼5 équi.). Après purification par HPLC préparative, 33 mg de produit 3 sont isolés. (rdt=43%). EIMS ([M+H]+): 369. RT= 3.70 min (gradient acétonitrile/eau de 5 à 85%- méthode A).

### Exemple 4 : Trifluoroacétate de 4-[(3-{[(2-méthoxyphényl) carbamoyl]amino}benzyl)amino]-1H-pyrazole-3-carboxamide

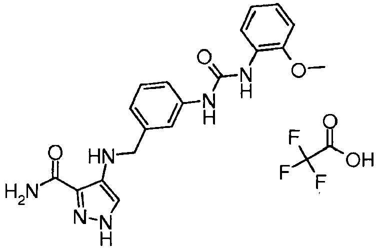

### Préparation de la 1-(2-Méthoxy-phényl)-3-(3-formyl-phényl)- urée :

Le produit a été préparé tel que décrit dans l'exemple 2 à partir de 484 mg de 3-aminobenzaldéhyde (4 mmoles) (forme polymérisée) et 597 mg de 2-méthoxy-phényl isocyanate (4 mmoles) dans 4 ml de DCE. 300 mg brut d'aldéhyde attendu sont isolés et directement utilisés pour l'étape suivante. ([M+H]+): 271. RT= 5.14 min (gradient acétonitrile/eau de 30 à 90%- méthode B).

### Préparation de l'exemple 4:

L'exemple 4 a été préparé selon la méthode décrite pour l'exemple 1 en partant de 200 mg de résine (ii), 162 mg de 1-(2-Méthoxy-phényl)-3-(3-formyl-phényl)-urée (0.6 mmole, 3 équi.) et 66 mg de cyanoborohydrure de sodium (1 mmole ; ∼5 équi.). Après purification par HPLC préparative, 10.4 mg de produit 4sont isolés. (rdt=11%). EIMS ([M+H]+): 381. RT= 3.84 min (gradient acétonitrile/eau de 5 à 85%- méthode A).

### Exemple 5: Trifluoroacétate de 4-{[3-({[2-fluoro-3-(trifluorométhyl) phényl]carbamoyl}amino)benzyl]amino}-1H-pyrazole-3-carboxamide

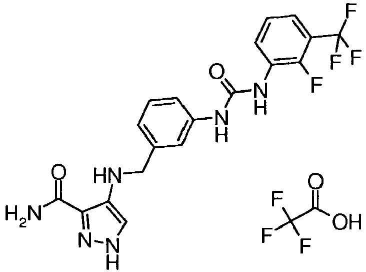

### Préparation de la 1-(2-Fluoro-3-trifluorométhyl-phényl)-3-(3-formyl-phényl)-urée :

Le produit a été préparé tel que décrit dans l'exemple 2 à partir de 484 mg de 3-aminobenzaldéhyde (4 mmoles) (forme polymérisée) et 820 mg de 2-fluoro-3-trifluorométhyl-phényl isocyanate (4 mmoles) dans 4 ml de DCE. 300 mg brut d'aldéhyde attendu sont isolés et directement utilisés pour l'étape suivante. ([M+H]+): 327. RT = 4.21 min (gradient acétonitrile/eau de 30 à 90%- méthode B).

### Préparation de l'exemple 5 :

L'exemple 5 a été préparé selon la méthode décrite pour l'exemple 1 en partant de 200 mg de résine (ii), 195 mg de 1-(2-Fluoro-3trifluorométhyl-phényl)-3-(3-formyl-phényl)-urée (0.6 mmole, 3 équi.) et 66 mg de cyanoborohydrure de sodium (1 mmole ; ∼5 équi.). Après purification par HPLC préparative, 31.4 mg de produit 5 sont isolés. (rdt=29%). EIMS ([M+H]+): 437. RT= 3.33 min (gradient acétonitrile/eau de 5 à 100%- méthode A).

### Exemple 6 : Trifluoroacétate de 4-[(3-{[(3-méthoxyphényl) carbamoyl]amino}benzyl)amino]-1H-pyrazole-3-carboxamide

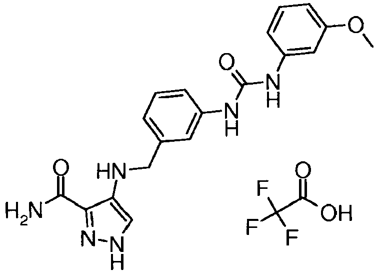

### Préparation de la 1-(3-méthoxy-phényl)-3-(3-formyl-phényl)- urée :

Le produit a été préparé tel que décrit dans l'exemple 2 à partir de 484 mg de 3-aminobenzaldéhyde (4 mmoles) (forme polymérisée) et 597 mg de 3-méthoxy-phényl isocyanate (4 mmoles) dans 4 ml de DCE. 598 mg d'aldéhyde brut attendu sont isolés et directement utilisés pour l'étape suivante. ([M+H]+): 271. RT= 5.05 min (gradient acétonitrile/eau de 30 à 90%- méthode B).

### Préparation de l'exemple 6:

L'exemple 6 a été préparé selon la méthode décrite pour l'exemple 1 en partant de 200 mg de résine (ii), 162 mg de 1-(3-Méthoxy-phényl)-3-(3-formyl-phényl)-urée (0.6 mmole, 3 équi.) et 66 mg de cyanoborohydrure de sodium (1 mmole ; ∼5 équi.). Après purification par HPLC préparative, 17 mg de produit 6 sont isolés. (rdt=17%). EIMS ([M+H]+): 381. RT= 2.47 min (gradient acétonitrile/eau de 5 à 100%- méthode A).

### Exemple 7: Trifluoroacétate de 4-{[3-({[3-fluoro-5-(trifluorométhyl) phényl]carbamoyl}amino)benzyl]amino}-1H-pyrazole-3-carboxamide

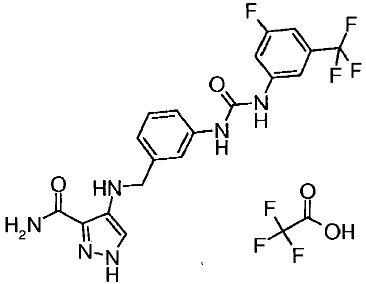

### Préparation de la 1-(3-Fluoro-5-trifluorométhyl-phényl)-3-(3-formyl-phényl)-urée :

Le produit a été préparé tel que décrit dans l'exemple 2 à partir de 484 mg de 3-aminobenzaldéhyde (4 mmoles) (forme polymérisée) et 597 mg de 3-fluoro-5-trifluorométhyl-phényl isocyanate (4 mmoles) dans 4 ml de DCE. 924 mg d'aldéhyde brut attendu sont isolés et directement utilisés pour l'étape suivante. ([M+H]+): 327. RT= 4.48 min (gradient acétonitrile/eau de 30 à 90%- méthode B).

### Préparation de l'exemple 7 :

L'exemple 7 a été préparé selon la méthode décrite pour l'exemple 1 en partant de 200 mg de résine (ii), 196 mg de 1-(3-Fluoro-5-trifluorométhyl-phényl)-3-(3-formyl-phényl)-urée (0.6 mmole, 3 équi.) et 66 mg de cyanoborohydrure de sodium (1 mmole ; ∼5 équi.). Après purification par HPLC préparative, 33.6 mg de produit 7 sont isolés. (rdt=31%). EIMS ([M+H]+): 437. RT= 3.55 min (gradient acétonitrile/eau de 5 à 100%- méthode A).

### Exemple 8 : Trifluoroacétate de 4-{[3-({[4-(trifluorométhoxy) phényl]carbamoyl}amino)benzyl]amino}-1H-pyrazole-3-carboxamide

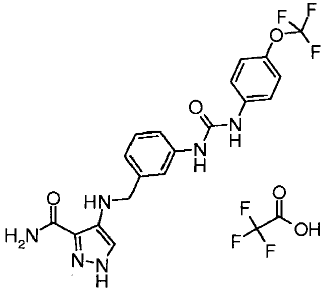

### Préparation de la 1-(4-Trifluorométhoxy-phényl)-3-(3-formyl-phényl)-urée :

Le produit a été préparé tel que décrit dans l'exemple 2 à partir de 484 mg de 3-aminobenzaldéhyde (4 mmoles) (forme polymérisée) et 812 mg de 4-trifluorométhoxy-phényl isocyanate (4 mmoles) dans 4 ml de DCE. 558 mg d'aldéhyde brut attendu sont isolés et directement utilisés pour l'étape suivante. ([M+H]+): 325. RT= 4.13 min (gradient acétonitrile/eau de 30 à 90%- méthode B).

### Préparation de l'exemple 8 :

L'exemple 8 a été préparé selon la méthode décrite pour l'exemple 1 en partant de 200 mg de résine (ii), 195 mg de 1-(4-trifluorométhoxy-phényl)-3-(3-formyl-phényl)-urée (0.6 mmole, 3 équi.) et 66 mg de cyanoborohydrure de sodium (1 mmole ; ~5 équi.). Après purification par HPLC préparative, 26.3 mg de produit 8 sont isolés. (rdt=24%). EIMS ([M+H]+): 435. RT= 3.34 min (gradient acétonitrile/eau de 5 à 100%- méthode A).

### Exemple 9: Trifluoroacétate de 3-{[(3-{[(3-carbamoyl-1H-pyrazol-4-yl)amino]méthyl}phényl)carbamoyl]amino}benzoate de méthyle

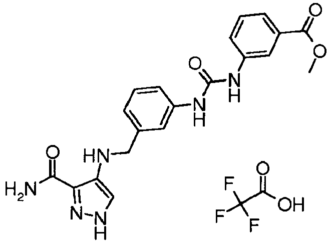

### Préparation de la 1-(3-Méthoxycarbonyl)-3-(3-Formyl-phényl)-urée

Le produit a été préparé tel que décrit dans l'exemple 2 à partir de 484 mg de 3-aminobenzaldéhyde (4 mmoles) (forme polymérisée) et 812 mg de l'ester méthylique de l'acide 3-Isocyano-benzoique (4 mmoles) dans 4 ml de DCE. 695 mg d'aldéhyde brut attendu sont isolés et directement utilisés pour l'étape suivante. ([M+H]+): 299. RT= 2.94 min (gradient acétonitrile/eau de 30 à 90%- méthode B).

### Préparation de l'exemple 9 :

L'exemple 9 a été préparé selon la méthode décrite pour l'exemple 1 en partant de 200 mg de résine (ii), 180 mg d'urée (0.6 mmole, 3 équi.) et 66 mg de cyanoborohydrure de sodium (1 mmole ; ~5 équi.). Après purification par HPLC préparative, 25.3 mg de produit 9 sont isolés. (rdt=24%). EIMS ([M+H]+): 409. RT= 2.67 min (gradient acétonitrile/eau de 5 à 100%- méthode A).

### Exemple 10 : Trifluoroacétate de 4-{[3-({[4-(trifluorométhyl) phényl]carbamoyl}amino)benzyl]amino}-1H-pyrazole-3-carboxamide

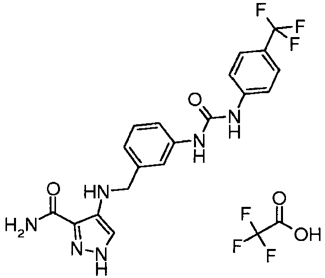

### Préparation : de la 1-(3-Formyl-phényl)-3-(4-trifluorométhyl-phényl)- urée :

Le produit a été préparé tel que décrit dans l'exemple 2 à partir de 484 mg de 3-aminobenzaldéhyde (4 mmoles) (forme polymérisée) et 748 mg de 4-trifluorométhyl-phényl isocyanate (4 mmoles) dans 4 ml de DCE. 896mg d'aldéhyde brut attendu sont isolés et directement utilisés pour l'étape suivante. ([M+H]+): 309. RT= 4.14 min (gradient acétonitrile/eau de 30 à 90%- méthode B).

### Préparation de l'exemple 10 :

L'exemple 10 a été préparé selon la méthode décrite pour l'exemple 1 en partant de 200 mg de résine (ii), 185 mg d'urée (0.6 mmole, 3 équi.) et 66 mg de cyanoborohydrure de sodium (1 mmole ; ~5 équi.). Après purification par HPLC préparative, 20.9 mg de produit 10 sont isolés. (rdt=19%). EIMS ([M+H]+): 419. RT= 3.33 min (gradient acétonitrile/eau de 5 à 100%- méthode A).

### Exemple 11 : Trifluoroacétate de 4-{[3-({[3-(trifluorométhyl) phényl]carbamoyl}amino)benzyl]amino}-1H-pyrazole-3-carboxamide

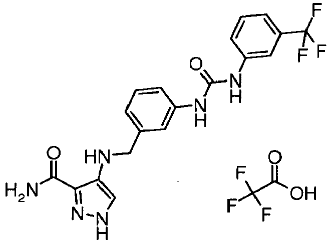

### Préparation : de la 1-(3-Formyl-phényl)-3-(3-trifluorométhyl-phényl)- urée :

Le produit a été préparé tel que décrit dans l'exemple 2 à partir de 484 mg de 3-aminobenzaldéhyde (4 mmoles) (forme polymérisée) et 748 mg de 3-trifluorométhyl-phényl isocyanate (4 mmoles) dans 4 ml de DCE. 744 mg d'aldéhyde brut attendu sont isolés et directement utilisés pour l'étape suivante. ([M+H]+): 309. RT= 4.04 min (gradient acétonitrile/eau de 30 à 90%- méthode B).

### Préparation de l'exemple 11 :

L'exemple 11 a été préparé selon la méthode décrite pour l'exemple 1 en partant de 200 mg de résine (ii), 185 mg d'urée (0.6 mmole, 3 équi.) et 66 mg de cyanoborohydrure de sodium (1 mmole ; ~5 équi.). Après purification par HPLC préparative, 25.9 mg de produit 11 sont isolés. (rdt=24%). EIMS ([M+H]+): 419. RT= 3.25 min (gradient acétonitrile/eau de 5 à 100%- méthode A).

### Exemple 12 : Trifluoroacétate de 4-{[3-({[2-(trifluorométhyl) phényl]carbamoyl}amino)benzyl]amino}-1H-pyrazole-3-carboxamide

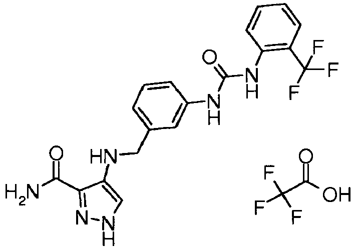

### Préparation : de la 1-(3-Formyl-phényl)-3-(2-trifluorométhyl-phényl)- urée :

Le produit a été préparé tel que décrit dans l'exemple 2 à partir de 484 mg de 3-aminobenzaldéhyde (4 mmoles) (forme polymérisée) et 748 mg de 2-trifluorométhyl-phényl isocyanate (4 mmoles) dans 4 ml de DCE. 744 mg d'aldéhyde brut attendu sont isolés et directement utilisés pour l'étape suivante. ([M+H]+): 309. RT= 5.51 min (gradient acétonitrile/eau de 30 à 90%- méthode B).

### Préparation de l'exemple 12:

L'exemple 12 a été préparé selon la méthode décrite pour l'exemple 1 en partant de 200 mg de résine (ii), 185 mg d'urée (0.6 mmole, 3 équi.) et 66 mg de cyanoborohydrure de sodium (1 mmole ; ~5 équi.). Après purification par HPLC préparative, 17.5 mg de produit 12 sont isolés. (rdt=16%). EIMS ([M+H]+): 419. RT= 2.64 min (gradient acétonitrile/eau de 5 à 100%- méthode A).

### Exemple 13 : Trifluoroacétate de 4-[(3-{[(3,5-diméthoxyphényl) carbamoyl]amino}benzyl)amino]-1H-pyrazole-3-carboxamide

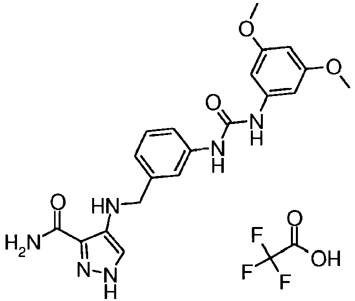

### Préparation : de la 1-(3-Formyl-phényl)-3-(3,5-dimethoxy-phényl)- urée :

Le produit a été préparé tel que décrit dans *l'exemple* 2 à partir de 484 mg de 3-aminobenzaldéhyde (4 mmoles) (forme polymérisée) et 717 mg de 3,5-diméthoxyphényl isocyanate (4 mmoles) dans 4 ml de DCE. 893 mg d'aldéhyde brut attendu sont isolés et directement utilisés pour l'étape suivante. ([M+H]+): 301. RT= 2.99 min (gradient acétonitrile/eau de 30 à 90%- méthode B).

### Préparation de l'exemple 13 :

L'exemple 13 a été préparé selon la méthode décrite pour l'exemple 1 en partant de 200 mg de résine (ii), 180 mg d'urée (0.6 mmole, 3 équi.) et 66 mg de cyanoborohydrure de sodium (1 mmole ; ~5 équi.). Après purification par HPLC préparative, 14.9 mg de produit 13 sont isolés. (rdt=14%). EIMS ([M+H]+): 411. RT= 4.61 min (gradient acétonitrile/eau de 5 à 85%- méthode A).

### Exemple 14 : Trifluoroacétate de 4-[(3-{[(3-méthylphényl) carbamoyl]amino}benzyl)amino]-1H-pyrazole-3-carboxamide

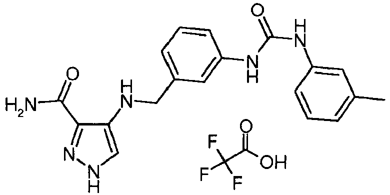

### Préparation : de la 1-(3-Formyl-phényl)- 3-(3-tolyl)-urée :

Le produit a été préparé tel que décrit dans l'exemple 2 à partir de 484 mg de 3-aminobenzaldéhyde (4 mmoles) (forme polymérisée) et 533 mg de 3-tolyl isocyanate (4 mmoles) dans 4 ml de DCE. 629 mg d'aldéhyde brut attendu sont isolés et directement utilisés pour l'étape suivante. ([M+H]+): 255. RT= 3.27 min (gradient acétonitrile/eau de 30 à 90%- méthode B).

### Préparation de l'exemple 14 :

L'exemple 14 a été préparé selon la méthode décrite pour l'exemple 1 en partant de 200 mg de résine (ii), 153 mg d'urée (0.6 mmole, 3 équi.) et 66 mg de cyanoborohydrure de sodium (1 mmole ; ~5 équi.). Après purification par HPLC préparative, 17 mg de produit 14 sont isolés. (rdt=18%). EIMS ([M+H]+): 365. RT= 4.66 min (gradient acétonitrile/eau de 5 à 85%- méthode B).

### Exemple 15 : Trifluoroacétate de 4-[(3-{[(4-méthoxyphényl) carbamoyl]amino}benzyl)amino]-1H-pyrazole-3-carboxamide

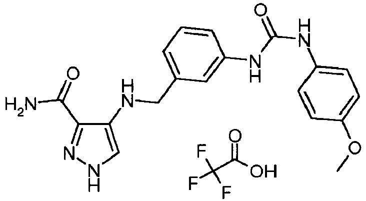

### Préparation : de la 1-(3-Formyl-phényl)-3-(4-méthoxy-phényl)-urée :

Le produit a été préparé tel que décrit dans l'exemple 2 à partir de 484 mg de 3-aminobenzaldéhyde (4 mmoles) (forme polymérisée) et 597 mg de 4-méthoxyphényl isocyanate (4 mmoles) dans 4 ml de DCE. 747 mg d'aldéhyde brut attendu sont isolés et directement utilisés pour l'étape suivante. ([M+H]+): 271. RT= 2.53 min (gradient acétonitrile/eau de 30 à 90%- méthode B).

### Préparation de l'exemple 15 :

L'exemple 15 a été préparé selon la méthode décrite pour l'exemple 1 en partant de 200 mg de résine (ii), 162 mg d'urée (0.6 mmole, 3 équi.) et 66 mg de cyanoborohydrure de sodium (1 mmole ; ~5 équi.). Après purification par HPLC préparative, 2.6 mg de produit 15sont isolés. (rdt=3%). EIMS ([M+H]+): 381. RT= 4.3 min (gradient acétonitrile/eau de 5 à 85%- méthode B).

### Exemple 16: Trifluoroacétate de 4-[(3-{[(4-fluorophényl) carbamoyl]amino}benzyl)amino]-1H-pyrazole-3-carboxamide

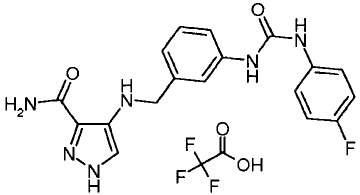

### Préparation : de la 1-(3-Formyl-phényl)-3-(4-fluoro-phényl)-urée :

Le produit a été préparé tel que décrit dans l'exemple 2 à partir de 484 mg de 3-aminobenzaldéhyde (4 mmoles) (forme polymérisée) et 548 mg de 4-fluorophényl isocyanate (4 mmoles) dans 4 ml de DCE. 760 mg d'aldéhyde brut attendu sont isolés et directement utilisés pour l'étape suivante. ([M+H]+): 259. RT= 2.86 min (gradient acétonitrile/eau de 30 à 90%- méthode B).

### Préparation de l'exemple 16 :

L'exemple 16 a été préparé selon la méthode décrite pour l'exemple 1 en partant de 200 mg de résine (ii), 155 mg d'urée (0.6 mmole, 3 équi.) et 66 mg de cyanoborohydrure de sodium (1 mmole ; ~5 équi.). Après purification par HPLC préparative, 0.6 mg de produit 16 sont isolés. (rdt=1%). EIMS ([M+H]+): 369. RT= 4.44 min (gradient acétonitrile/eau de 5 à 85%- méthode B).

### Exemple 17: Trifluoroacétate de 4-{[3-({[4-chloro-3-(trifluorométhyl) phényl]carbamoyl}amino)benzyl]amino}-1H-pyrazole-3-carboxamide

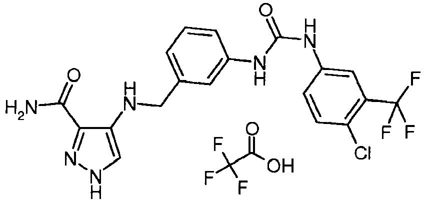

### Préparation : de la 1-(3-Formyl-phényl)- 3-(3-trifluoro-4-chloro-phényl) urée :

Le produit a été préparé tel que décrit dans l'exemple 2 à partir de 484 mg de 3-aminobenzaldéhyde (4 mmoles) (forme polymérisée) et 886 mg de 4-chloro-3-trifluorofluoro-phényl isocyanate (4 mmoles) dans 4 ml de DCE. 117 mg d'aldéhyde brut attendu sont isolés et directement utilisés pour l'étape suivante. ([M+H]+): 343. RT= 4.66 min (gradient acétonitrile/eau de 30 à 90%- méthode B).

### Préparation de l'exemple 17 :

L'exemple 17a été préparé selon la méthode décrite pour l'exemple 1 en partant de 200 mg de résine (ii), 206 mg d'urée (0.6 mmole, 3 équi.) et 66 mg de cyanoborohydrure de sodium (1 mmole ; ~5 équi.)., Après purification par HPLC préparative, 7.3 mg de produit 17 sont isolés. (rdt=6%). EIMS ([M+H]+): 453. RT= 5.38 min (gradient acétonitrile/eau de 5 à 85%- méthode B).

### Exemple 18: Trifluoroacétate de 4-{[3-({[4-(difluorométhoxy) phényl]carbamoyl}amino)benzyl]amino}-1H-pyrazole-3-carboxamide

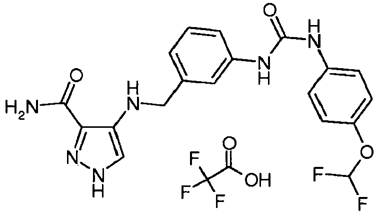

### Préparation : de la 1-(3-Formyl-phényl)- 3-(4-difluorométhoxy-phényl) urée :

Le produit a été préparé tel que décrit dans l'exemple 2 à partir de 484 mg de 3-aminobenzaldéhyde (4 mmoles) (forme polymérisée) et 741 mg de 4-difluorométhoxy-phényl isocyanate (4 mmoles) dans 4 ml de DCE. 840 mg d'aldéhyde brut attendu sont isolés et directement utilisés pour l'étape suivante. ([M+H]+): 307. RT= 3.39 min (gradient acétonitrile/eau de 30 à 90%- méthode B).

### Préparation de l'exemple 18 :

L'exemple 18 a été préparé selon la méthode décrite pour l'exemple 1 en partant de 200 mg de résine (ii), 184 mg d'urée (0.6 mmole, 3 équi.) et 66 mg de cyanoborohydrure de sodium (1 mmole ; ~5 équi.). Après purification par HPLC préparative, 28.5 mg de produit 18 sont isolés. (rdt=27%). EIMS ([M+H]+): 417. RT= 4.9 min (gradient acétonitrile/eau de 5 à 85%- méthode B).

### Exemple 19 : Trifluoroacétate de 4-{[3-({[2-chloro-4-(trifluorométhyl)phényl]carbamoyl}amino)benzyl]amino}-1H-pyrazole-3-carboxamide

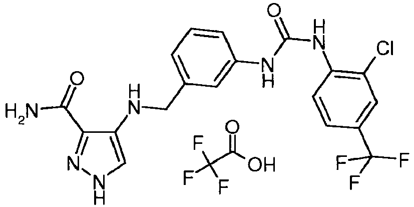

### Préparation : de la 1-(3-Formyl-phényl)-3-(2-chloro-4-trifluorométhyl-phényl) urée :

Le produit a été préparé tel que décrit dans l'exemple 2 à partir de 484 mg de 3-aminobenzaldéhyde (4 mmoles) (forme polymérisée) et 886 mg de 2-cholro-4-trifluorométhyl -phényl isocyanate (4 mmoles) dans 4 ml de DCE. 1 g d'aldéhyde brut attendu sont isolés et directement utilisés pour l'étape suivante. ([M+H]+): 343. RT = 4.66 min (gradient acétonitrile/eau de 30 à 90%- méthode B).

### Préparation de l'exemple 19:

L'exemple 19 a été préparé selon la méthode décrite pour l'exemple 1 en partant de 200 mg de résine (ii), 206 mg d'urée (0.6 mmole, 3 équi.) et 66 mg de cyanoborohydrure de sodium (1 mmole ; ∼5 équi.). Après purification par HPLC préparative, 24.3 mg de produit 19 sont isolés. (rdt=22%). EIMS ([M+H]+): 453. RT= 5.36 min (gradient acétonitrile/eau de 5 à 85%- méthode B).

### Exemple 20 : Trifluoroacétate de 4-[(3-{[(4-méthylphényl) carbamoyl]amino}benzyl)amino]-1H-pyrazole-3-carboxamide

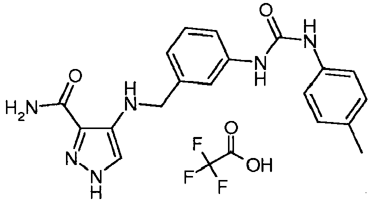

### Préparation : de la 1-(3-Formyl-phényl)- 3-(4-tolyl)-urée :

Le produit a été préparé tel que décrit dans l'exemple 2 à partir de 484 mg de 3-aminobenzaldéhyde (4 mmoles) (forme polymérisée) et 533 mg de 4-tolyl isocyanate (4 mmoles) dans 4 ml de DCE. 683 mg d'aldéhyde brut attendu sont isolés et directement utilisés pour l'étape suivante. ([M+H]+): 255. RT = 3.16 min (gradient acétonitrile/eau de 30 à 90%- méthode B).

### Préparation de l'exemple 20:

L'exemple 20 a été préparé selon la méthode décrite pour l'exemple 1 en partant de 200 mg de résine (ii), 153 mg d'urée (0.6 mmole, 3 équi.) et 66 mg de cyanoborohydrure de sodium (1 mmole ; ~5 équi.). Après purification par HPLC préparative, 15.3 mg de produit 20 sont isolés. (rdt=16%). EIMS ([M+H]+): 365. RT= 4.58 min (gradient acétonitrile/eau de 5 à 85%- méthode B).

### Exemple 21 : Trifluoroacétate de 4-[(3-{[(2,5-diméthylphényl) carbamoyl]amino}benzyl)amino]-1H-pyrazole-3-carboxamide (

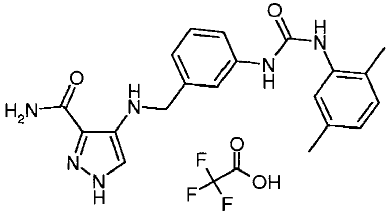

### Préparation : de la 1-(3-Formyl-phényl)- 3-(2,5-diméthyl-phényl) urée :

Le produit a été préparé tel que décrit dans l'exemple 2 à partir de 484 mg de 3-aminobenzaldéhyde (4 mmoles) (forme polymérisée) et 589 mg 2,5-diméthyl-phényl isocyanate (4 mmoles) dans 4 ml de DCE. 660 mg d'aldéhyde brut attendu sont isolés et directement utilisés pour l'étape suivante. ([M+H]+): 269. RT= 3.39 min (gradient acétonitrile/eau de 30 à 90%- méthode B).

### Préparation de l'exemple 21 :

L'exemple 21 a été préparé selon la méthode décrite pour l'exemple 1 en partant de 200 mg de résine (ii), 161 mg d'urée (0.6 mmole, 3 équi.) et 66 mg de cyanoborohydrure de sodium (1 mmole ; ∼5 équi.). Après purification par HPLC préparative, 26.7 mg de produit 21 sont isolés. (rdt=27%). EIMS ([M+H]+): 379. RT= 4.68 min (gradient acétonitrile/eau de 5 à 85%- méthode B).

### Exemple 22 : Trifluoroacétate de 4-[(3-{[(3,4-diméthylphényl) carbamoyl]amino}benzyl)amino]-1H-pyrazole-3-carboxamide

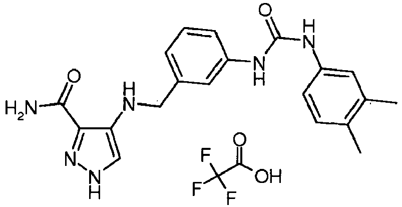

### Préparation : de la 1-(3-Formyl-phényl)- 3-(3,4-diméthyl-phényl)-urée :

Le produit a été préparé tel que décrit dans l'exemple 2 à partir de 484 mg de 3-aminobenzaldéhyde (4 mmoles) (forme polymérisée) et 589 mg 3,4-diméthyl-phényl isocyanate (4 mmoles) dans 4 ml de DCE. 621 mg d'aldéhyde brut attendu sont isolés et directement utilisés pour l'étape suivante. ([M+H]+): 269. RT= 3.55 min (gradient acétonitrile/eau de 30 à 90%- méthode B).

### Préparation de l'exemple 22 :

L'exemple 22 a été préparé selon la méthode décrite pour l'exemple 1 en partant de 200 mg de résine (ii), 161 mg d'urée (0.6 mmole, 3 équi.) et 66 mg de cyanoborohydrure de sodium (1 mmole ; ∼5 équi.). Après purification par HPLC préparative, 32 mg de produit 22 sont isolés. (rdt=32%). EIMS ([M+H]+): 379. RT= 4.8 min (gradient acétonitrile/eau de 5 à 85%- méthode B).

### Exemple 23: Trifluoroacétate de 4-[(3-{[(2-méthylphényl) carbamoyl]amino}benzyl)amino]-1H-pyrazole-3-carboxamide

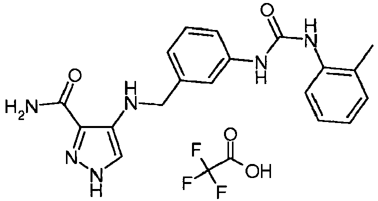

### Préparation : de la 1-(3-Formyl-phényl)- 3-(2-tolyl) urée :

Le produit a été préparé tel que décrit dans l'exemple 2 à partir de 484 mg de 3-aminobenzaldéhyde (4 mmoles) (forme polymérisée) et 533 mg 2-Tolyl isocyanate (4 mmoles) dans 4 ml de DCE. 621 mg d'aldéhyde brut attendu sont isolés et directement utilisés pour l'étape suivante. ([M+H]+): 255. RT= 5.14 min (gradient acétonitrile/eau de 30 à 90%- méthode B).

### Préparation de l'exemple 23 :

L'exemple 23 a été préparé selon la méthode décrite pour l'exemple 1 en partant de 200 mg de résine (ii), 153 mg d'urée (0.6 mmole, 3 équi.) et 66 mg de cyanoborohydrure de sodium (1 mmole ; ∼5 équi.). Après purification par HPLC préparative, 16.7 mg de produit 23 sont isolés. (rdt=17%). EIMS ([M+H]+): 365. RT= 4.5 min (gradient acétonitrile/eau de 5 à 85%- méthode B).

### Exemple 24: Trifluoroacétate de 4-[(3-{[(3-éthylphényl) carbamoyl]amino}benzyl)amino]-1H-pyrazole-3-carboxamide

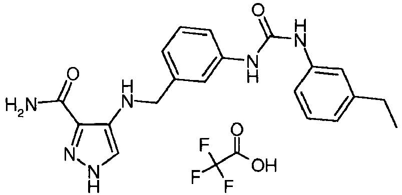

### Préparation : de la 1-(3-Formyl-phényl)- 3-(3-éthyl-phényl) urée :

Le produit a été préparé tel que décrit dans l'exemple 2 à partir de 484 mg de 3-aminobenzaldéhyde (4 mmoles) (forme polymérisée) et 589 mg 3-éthyl-phényle isocyanate (4 mmoles) dans 4 ml de DCE. 732 mg d'aldéhyde brut attendu sont isolés et directement utilisés pour l'étape suivante. ([M+H]+): 255. RT= 5.51 min (gradient acétonitrile/eau de 30 à 90%- méthode B).

### Préparation de l'exemple 24 :

L'exemple 24 a été préparé selon la méthode décrite pour l'exemple 1 en partant de 200 mg de résine (ii), 160 mg d'urée (0.6 mmole, 3 équi.) et 66 mg de cyanoborohydrure de sodium (1 mmole ; ∼5 équi.). Après purification par HPLC préparative, 12.2 mg de produit 24 sont isolés. (rdt=12%). EIMS ([M+H]+): 365. RT= 4.5 min (gradient acétonitrile/eau de 5 à 85%- méthode B).

### Exemple 25: Trifluoroacétate de 4-{[3-({[3,5-bis(trifluorométhyl) phényl]carbamoyl}amino)benzyl]amino}-1H-pyrazole-3-carboxamide

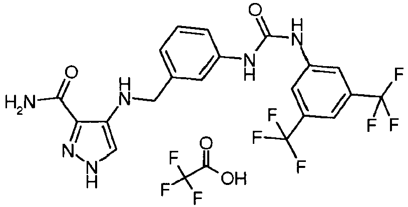

### Préparation de la 1-(3-Formyl-phényl)- 3-(3,5-bis-trifluorométhyl-phényl) urée :

Le produit a été préparé tel que décrit dans l'exemple 2 à partir de 484 mg de 3-aminobenzaldéhyde (4 mmoles) (forme polymérisée) et 1.02 g 3,5-bis-trifluorométhyl-phényle isocyanate (4 mmoles) dans 4 ml de DCE. 489 mg d'aldéhyde brut attendu sont isolés et directement utilisés pour l'étape suivante. ([M+H]+): 377. RT= 5.05 min (gradient acétonitrile/eau de 30 à 90%- méthode B).

### Préparation de l'exemple 25 :

L'exemple 25 a été préparé selon la méthode décrite pour *l'exemple* 1 en partant de 200 mg de résine (ii), 225 mg d'urée (0.6 mmole, 3 équi.) et 66 mg de cyanoborohydrure de sodium (1 mmole ; ∼5 équi.). Après purification par HPLC préparative, 3.3 mg de produit 25 sont isolés. (rdt=4%). EIMS ([M+H]+): 365. RT= 4.5 min (gradient acétonitrile/eau de 5 à 85%- méthode B).

### Exemple 26: Trifluoroacétate de 4-[(3-{[(3-fluorophényl) carbamoyl]amino}benzyl)amino]-1H-pyrazole-3-carboxamide

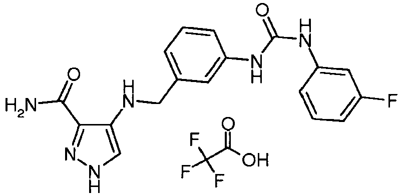

### Préparation de la 1-(3-Formyl-phényl)- 3-(3-fluoro-phényl) urée :

Le produit a été préparé tel que décrit dans l'exemple 2 à partir de 484 mg de 3-aminobenzaldéhyde (4 mmoles) (forme polymérisée) et 548 mg 3-fluoro-phényle isocyanate (4 mmoles) dans 4 ml de DCE. 723 mg d'aldéhyde brut attendu sont isolés et directement utilisés pour l'étape suivante. ([M+H]+): 259. RT= 4.04 min (gradient acétonitrile/eau de 30 à 90%- méthode B).

### Préparation de l'exemple 26 :

L'exemple 26 a été préparé selon la méthode décrite pour l'exemple 1 en partant de 200 mg de résine (ii), 155 mg d'urée (0.6 mmole, 3 équi.) et 66 mg de cyanoborohydrure de sodium (1 mmole ; ∼5 équi.). Après purification par HPLC préparative, 23.5 mg de produit 26 sont isolés. (rdt=25%). EIMS ([M+H]+): 369. RT= 4.7 min (gradient acétonitrile/eau de 5 à 85%- méthode B).

### Exemple 27 : Trifluoroacétate de 4-[(3-{[(2-méthoxy-5-méthylphényl)carbamoyl]amino}benzyl)amino]-1H-pyrazole-3-carboxamide

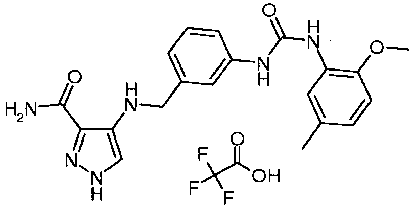

### Préparation de la 1-(3-Formyl-phényl)- 3-(2-méthoxy-5-méthyl-phényl) urée :

Le produit a été préparé tel que décrit dans l'exemple 2 à partir de 484 mg de 3-aminobenzaldéhyde (4 mmoles) (forme polymérisée) et 653 mg de 2-Méthoxy-5-méthyl-phényle isocyanate (4 mmoles) dans 4 ml de DCE. 797 mg d'aldéhyde brut attendu sont isolés et directement utilisés pour l'étape suivante. ([M+H]+): 285. RT=2.94 min (gradient acétonitrile/eau de 30 à 90%- méthode B).

### Préparation de l'exemple 27:

L'exemple 27 a été préparé selon la méthode décrite pour l'exemple 1 en partant de 200 mg de résine (ii), 170 mg d'urée (0.6 mmole, 3 équi.) et 66 mg de cyanoborohydrure de sodium (1 mmole ; ∼5 équi.). Après purification par HPLC préparative, 20 mg de produit 27 sont isolés. (rdt=25%). EIMS ([M+H]+): 395. RT= 5 min (gradient acétonitrile/eau de 5 à 85%- méthode B).

### Exemple 28 : Trifluoroacétate de 4-[(3-{[(2,5-diméthoxyphényl) carbamoyl]amino}benzyl)amino]-1H-pyrazole-3-carboxamide

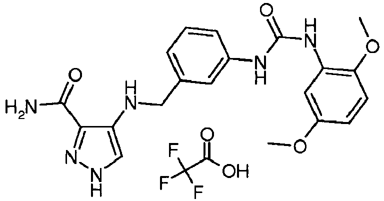

### Préparation de la 1-(3-Formyl-phényl)- 3-(2,5-diméthoxy -phényl) urée :

Le produit a été préparé tel que décrit dans l'exemple 2 à partir de 484 mg de 3-aminobenzaldéhyde (4 mmoles) (forme polymérisée) et 717 mg de 2,5-Diméthoxy - phényle isocyanate (4 mmoles) dans 4 ml de DCE. 853 mg d'aldéhyde brut attendu sont isolés et directement utilisés pour l'étape suivante. ([M+H]+): 301. RT= 4.14 min (gradient acétonitrile/eau de 30 à 90%- méthode B).

### Préparation de l'exemple 28 :

L'exemple 28 a été préparé selon la méthode décrite pour l'exemple 1 en partant de 200 mg de résine (ii), 170 mg d'urée (0.6 mmole, 3 équi.) et 66 mg de cyanoborohydrure de sodium (1 mmole ; ∼5 équi.). Après purification par HPLC préparative, 11.8 mg de produit 28 sont isolés. (rdt=12%). EIMS ([M+H]+): 411. RT= 4.72 min (gradient acétonitrile/eau de 5 à 85%- méthode B).

### Exemple 29 : Trifluoroacétate de 4-{[3-({[3-chloro-4-(difluorométhoxyphényl]carbamoyl}amino)benzyl]amino}-1H-pyrazole-3-carboxamide

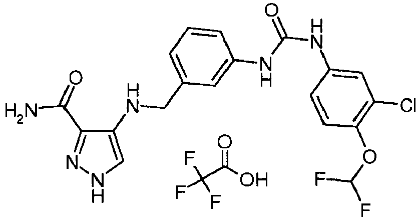

### Préparation de la 1-(3-Formyl-phényl)- 3-(3-chloro-4-difluorométhoxy -phényl) urée :

Le produit a été préparé tel que décrit dans l'exemple 2 à partir de 484 mg de 3-aminobenzaldéhyde (4 mmoles) (forme polymérisée) et 878 mg de 3-chloro-4-difluorométhoxy -phényle isocyanate (4 mmoles) dans 4 ml de DCE. 936 mg d'aldéhyde brut attendu sont isolés et directement utilisés pour l'étape suivante. ([M+H]+): 341. RT= 4.21 min (gradient acétonitrile/eau de 30 à 90%- méthode B).

### Préparation de l'exemple 29 :

L'exemple 29 a été préparé selon la méthode décrite pour l'exemple 1 en partant de 200 mg de résine (ii), 204 mg d'urée (0.6 mmole, 3 équi.) et 66 mg de cyanoborohydrure de sodium (1 mmole ; ∼5 équi.). Après purification par HPLC préparative, 8.3 mg de produit 29 sont isolés. (rdt=7%). EIMS ([M+H]+): 451. RT= 5.36 min (gradient acétonitrile/eau de 5 à 85%- méthode B).

### Exemple 30 : Trifluoroacétate de 4-[(3-{[(2,5-difluorophényl) carbamoyl]amino}benzyl)amino]-1H-pyrazole-3-carboxamide

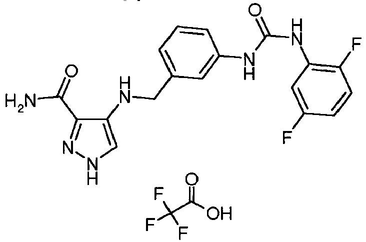

### Préparation de la 1-(3-Formyl-phényl)- 3-(2,5-difluoro-phényl) urée :

Le produit a été préparé tel que décrit dans l'exemple 2 à partir de 484 mg de 3-aminobenzaldéhyde (4 mmoles) (forme polymérisée) et 620 mg de 2,5-Difluoro-phényle isocyanate (4 mmoles) dans 4 ml de DCE. 952 mg d'aldéhyde brut attendu sont isolés et directement utilisés pour l'étape suivante. ([M+H]+): 277. RT= 4.13 min (gradient acétonitrile/eau de 30 à 90%- méthode B).

### Préparation de l'exemple 30 :

L'exemple 30 a été préparé selon la méthode décrite pour l'exemple 1 en partant de 200 mg de résine (ii), 167 mg d'urée (0.6 mmole, 3 équi.) et 66 mg de cyanoborohydrure de sodium (1 mmole ; ∼5 équi.). Après purification par HPLC préparative, 14.1 mg de produit 30sont isolés. (rdt=14%). EIMS ([M+H]+): 387. RT= 4.82 min (gradient acétonitrile/eau de 5 à 85%- méthode B).

### Exemple 31 : Trifluoroacétate de 4-{[3-({[4-méthyl-3-(trifluorométhyl)phényl]carbamoyl}amino)benzyl]amino}-1H-pyrazole-3-carboxamide

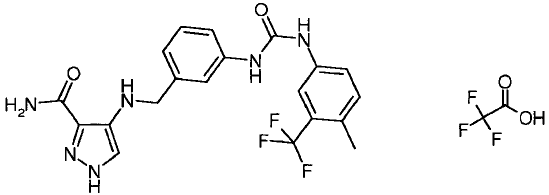

### Préparation de la 1-(3-Formyl-phényl)- 3-(4-méthyl-3-trifuorométhyl-phényl) urée

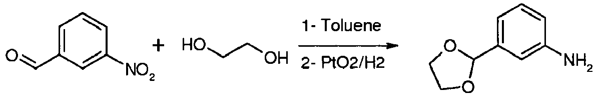

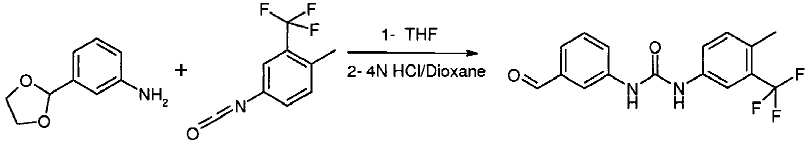

L'urée a été préparée de la façon suivante : 3 g de 3-nitrobenzaldehyde (20 mmoles), 3.4 ml d'éthylène glycol (60 mmoles) et 0.3 g d'acide para-toluène sulphonique en solution dans 250ml de toluène sont portés à ébullition pendant 4 heures puis le mélange est versé dans 100 ml de solution saturée de bicarbonate de sodium et extrait avec 2 fois 50 ml d'acétate d'éthyle. Les phases organiques rassemblées sont lavées à l'eau, séchées sur sulfate de sodium et évaporées sous vide. Le brut est directement hydrogéné dans 20 ml de THF en présence de160 mg d'oxyde de platine dans un appareil de Parr. Après 4 heures d'hydrogénation à température ambiante, le mélange réactionnel est filtré sur célite. Pour éviter toute dégradation l'aniline obtenue est laissée en solution dans le THF à la concentration de 10 mmoles/20ml et utilisée ainsi pour la formation d'urée.
2 ml de la solution d'aniline (1 mmole) est traitée avec 200 mg de 4-Méthyle-3-trifluorométhyle-phényle isocyanate pendant 4 heures à température ambiante. Le mélange est versé dans 100 ml de solution d'HCl à 10% et extrait avec 2 fois 50 ml d'acétate d'éthyle. Les phases organiques rassemblées sont lavées à l'eau, sèchées sur sulfate de sodium et évaporées sous vide. On isole 320 mg de produit attendu sous forme de solide. (rdt quantitatif) EIMS ([M+H]+): 323. RT= 4.37 min (gradient acétonitrile/eau de 30 à 90%- méthode B).

### Préparation de l'exemple 31 :

L'exemple 31 a été préparé selon la méthode décrite pour l'exemple 1 en partant de 200 mg de résine (ii), 193 mg d'urée (0.6 mmole, 3 équi.) et 66 mg de cyanoborohydrure de sodium (1 mmole ; ∼5 équi.). Après purification par HPLC préparative, 36.7 mg de produit 31 sont isolés. (rdt=34%). EIMS ([M+H]+): 433. RT= 4.64 min (gradient acétonitrile/eau de 5 à 85%- méthode B).

### Exemple 32: Trifluoroacétate de 4-{[3-({[2-fluoro-5-(trifluorométhyl)phényl]carbamoyl}amino)benzyl]amino}-1H-pyrazole-3-carboxylate de méthyle

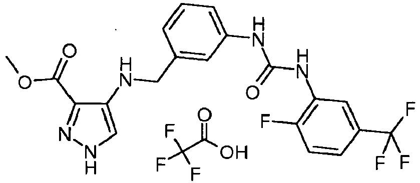

Le composé 32 a été préparé par directe amination réductrice de l'acide 4-amino-3-pyrazole carboxylique méthyle ester. Une solution de 44 mg de l'acide 4-amino-3-pyrazole carboxylique méthyle ester (0.31 mmole) et 100 mg de la 1-(2-Fluoro-5-trifluorométhyl-phényl)-3-(3-formyl-phényl) urée (voir exemple 2) dans un mélange de 0.6 ml de DCE et 0.5 ml de DMF est traitée par une solution de 59 mg de cyanoborohydrure de sodium dans 0.5 ml de méthanol et 0.05 ml d'acide acétique. Le mélange est agité 2 heures à 80°C puis refroidi et versé dans 20 ml d'eau. Le mélange est extrait avec 2 fois 20 ml d'acétate d'éthyle. Les phases organiques rassemblées sont lavées à l'eau, séchées sur sulfate de sodium et évaporées sous vide. Après purification par HPLC préparative, 20.8 mg de produit 32 sont isolés. (rdt=12%). EIMS ([M+H]+): 452. RT= 5.52 min (gradient acétonitrile/eau de 5 à 85%- méthode B).

### Exemple 33: Trifluoroacétate de 4-(1-{3-[3-(2-fluoro-5-trifluorométhyl-phényl)uréido]-phényl-éthylamino}-1H-pyrazole-3-carboxamide (1 :1)

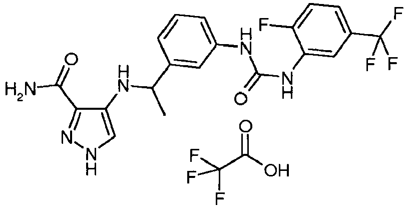

### Préparation de la 1-(3-acétyl-phényl)-3-(2-fluoro-5-trifluorométhyl-phényl)-urée:

Un mélange de 181 mg de 3-aminoacétophénone (1.34 mmole) et 275 mg de 2-fluoro-5-trifluorométhyl-phényl isocyanate (1.34 mmole) dans 1 ml de THF est laissé sous agitation à température ambiante pendant 1 heure, puis évaporé. Le solide est repris par de l'éther et filtré. On isole 307 mg de cétone attendue (rdt= 69%) avec une pureté LC/MS de 87%. Le produit brut est directement utilisé pour l'étape suivante. ([M+H]+): 341. Rét. Time: 6.06 min (méthode A).

### Préparation de l'exemple 33 :

450 mg de résine I (0.45 mmol) est gonflée dans 2 ml de DCE, puis 307 mg de 1-(3-acétyl-phényl)-3-(2-fluoro-5-trifluorométhyl-phényl)-urée (0.9 mmoles ; 2 équi.) en solution dans 2 ml de DMF sont additionnés suivis de 149 mg de cyanoborohydrure de sodium (2.25 mmoles ; 5 équi.). Le mélange est traité dans un four à micro-onde CEM Discover à 100°C pendant 10 minutes (puissance 90) La résine est ensuite lavée successivement avec 2 fois 2 ml de MeOH, 3 fois 2 ml de dichlorométhane, 2 fois 2 ml de MeOH et 3 fois 2 ml de dichlorométhane. Le produit est clivé par traitement de la résine avec 4 ml d'une solution d'acide trifluoroacétique/dichlorométhane à 50/50. La solution est évaporée et le brut obtenu est directement purifié par HPLC préparative. Après lyophilisation, 13.5 mg de produit attendu est obtenu (solide blanc, rdt=5%). ([M+H]+): 451). RT: 4.77 min (Méthode A)

### Exemple 34: 4-({3-[3-(2-fluoro-5-trifluorométhyl-phényl)-uréido]-benzyl}-méthyl-amino)-1h-pyrazole-3-carboxamide

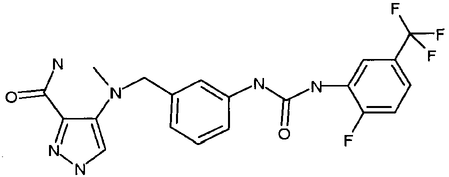

A une solution de 0.25g de 4-[(3-amino-benzyl)-méthyl-amino]-1 H-pyrazole-3-carboxamide dans 25ml de THF anhydre on ajoute à 20°C une solution de 0.14ml de 1-fluoro-2-isocyanato-4-trifluorométhyl-benzène. Le milieu réactionnel est agité 12h à 20°C, puis dilué par 100 ml d'acétate d'éthyle. La phase organique est lavée par 50ml d'eau distillée, puis décantée, séchée sur sulfate de magnésium et évaporée à sec sous pression réduite. Le résidu obtenu est chromatographié sur colonne de silice (15g de silice cartouche Merck de granulométrie de 15 à 45µm, diamètre de colonne 2.2 cm, fractions de 5ml, débit de 10ml/min, éluant dichlorométhane 95 méthanol 5 en volumes). Les fractions 35 à 95 sont réunies, évaporées à sec sous pression réduite. On obtient 0.02g de 4-({3-[3-(2-fluoro-5-trifluorométhyl-phényl)-uréido]-benzyl}-méthyl-amino)-1h-pyrazole-3-carboxamide sous la forme d'un solide. ([M+H]+): 451). RT: 3.55 min (Méthode A)

Le 4-[(3-amino-benzyl)-méthyl-amino]-1H-pyrazole-3-carboxamide est obtenu de la façon suivante :

A une solution de 1.26g de 4-[méthyl-(3-nitro-benzyl)-amino]-1*H*-pyrazole-3-carboxamide dans 90 ml d'éthanol absolu on ajoute par petites portions 3.6g de chlorure d'étain dihydrate à 20°C. Le milieu réactionnel est agité 15h à 20°C, puis amené à sec sous pression réduite. Le résidu est repris par 500ml d'un mélange chlorure de méthylène 90 / méthanol 10 (en volumes) et 500 ml d'une solution aqueuse saturée en hydrogénocarbonate de potassium. Ce milieu est agité à 20°C pendant 2h, puis filtré. Le solide obtenu après filtration est extrait deux fois par un mélange de 100ml de chlorure de méthylène 90 méthanol 10 (en volumes), les phases liquides sont réunies, décantées. La phase aqueuse est extraite par 2 fois 200ml de chlorure de méthylène, les phases organiques sont réunies, lavées par 300ml d'une solution aqueuse saturée en chlorure de sodium, séchées sur sulfate de sodium, filtrées et évaporées à sec sous pression réduite. On obtient 0.6g de 4-[(3-amino-benzyl)-méthyl-amino]-1 H-pyrazole-3-carboxamide sous la forme d'une meringue crème. ([M+H]+): 246). RT:0.3 min

### (Méthode A)

Le 4-[méthyl-(3-nitro-benzyl)-amino]-1*H*-pyrazole-3-carboxamide est préparé de la façon suivante :

A une solution de 2.49g de 4-[méthyl-(3-nitro-benzyl)-amino]-1*H*-pyrazole-3-carboxylic acid 2,4-diméthoxy-benzylamide dans 30ml de toluène on ajoute 4.45g d'acide paratoluènesulfonique monohydrate, puis on chauffe le milieu réactionnel au reflux du toluène pendant 17h. Après refroidissement à température ambiante du milieu réactionnel, on ajoute 15ml de méthanol, puis 700ml d'acétate d'éthyle, puis enfin 300ml d'eau distillée. On ajuste le pH de ce milieu à une valeur de 11 par ajout de 100ml d'une solution aqueuse 1 N de soude. Cette solution est filtrée, le solide est extrait deux fois par 30ml d'acétate d'éthyle, les phases liquides sont réunies, puis décantées. La phase aqueuse est extraite par 2 fois 200ml de chlorure de méthylène, les phases organiques sont réunies, lavées par 300ml d'une solution aqueuse saturée en chlorure de sodium, séchées sur sulfate de magnésium, filtrées et évaporées à sec sous pression réduite. On obtient 1.41g de 4-[méthyl-(3-nitro-benzyl)-amino]-1*H*-pyrazole-3-carboxamide sous la forme d'un solide beige fondant à 166°C. ([M+H]+): 276). RT: 2.61 min (Méthode A)

Le 4-[méthyl-(3-nitro-benzyl)-amino]-1*H*-pyrazole-3-carboxylic acid 2,4-diméthoxy-benzylamide est obtenu de la façon suivante :

Une solution de 1.61g de 4-(3-nitro-benzylamino)-1 H-pyrazole-3-carboxylic acid 2,4-dimethoxy-benzylamide, 1.25g de paraformaldéhyde et 0.94g de sulfate de magnésium anhydre dans 70ml d'acide acétique glacial est agitée à température ambiante pendant 4h. On ajoute ensuite à cette solution 1.23g de cyanoborohydrure de sodium par petites fractions. Le milieu réactionnel est agité 2h à température ambiante, puis coulé sur 300ml d'une solution aqueuse de soude 5N et 120g de glace pilée en ajustant le pH à 11. On ajoute ensuite 300ml d'une solution saturée en chlorure de sodium. Cette solution est filtrée, le solide lavé trois fois par 60ml d'eau distillée. Le solide ainsi recueilli est séché à l'air. On obtient 1.51g de 4-[méthyl-(3-nitro-benzyl)-amino]-1*H*-pyrazole-3-carboxylic acid 2,4-diméthoxy-benzylamide sous la forme d'un solide jaune pâle. ([M+H]+): 426). RT: 3.87 min (Méthode A)

Le 4-(3-nitro-benzylamino)-1H-pyrazole-3- carboxylique-acide-2.4-diméthoxy benzylamide est obtenu de la façon suivante :

A une solution de 15.43g de_4-amino-1-H-pyrazole-3-carboxylique acid-2,4-diméthoxy-benzylamide chlorhydrate et 7.01g de diisopropyléthylamine dans 490ml de tétrahydrofuranne anhydre on ajoute 8.2g de 3-nitro-benzaldéhyde et 5.9g de sulfate de magnésium anhydre. Le milieu réactionnel est chauffé au reflux pendant 1h30, laissé revenir à 20°C puis refroidi à 5°C à l'aide d'un bain eau glacée. On ajoute à la suspension crème obtenue, par petites portions 15.5g de cyanoborohydrure de sodium. Le milieu réactionnel est agité 5 minutes à 5°C puis on laisse revenir à température ambiante. Laisser agiter à température ambiante pendant 20heures.La solution trouble orangé-marron obtenue est coulée sur 1500ml eau distillée. On ajoute 1000ml de dichlorométhane à la solution laiteuse marron clair obtenue. Après agitation, puis décantation de la phase chlorométhylénique, on extrait de nouveau la phase aqueuse avec deux fois 500ml de dichlorométhane .Les phases organiques sont réunies puis lavées avec 500ml d'une solution aqueuse saturée de chlorure de sodium, séchées sur sulfate de magnésium, filtrées sur papier, puis amenées à sec à l'évaporateur rotatif (Temp. 40°C P : 15mbar). On obtient 27.19g d'un solide jaune marron collant qui est recristallisé dans 360 ml d'acétonitrile au reflux. On obtient un premier lot de 7.7g de 4-(3-nitro-benzylamino)-1 H-pyrazole-3- carboxylique-acide-2.4-diméthoxy benzylamide sous la forme d'un solide jaune.

Le filtrat acétonitrile est récupéré puis amené à sec à l'évaporateur rotatif (temp.40°C P : 15mbar). On obtient 19.7g d'une masse collante ocre qui est triturée à 20°C avec 50ml d'acétonitrile pendant 1 heure. La suspension obtenue est filtrée sur VF n°3, lavée avec deux fois 15ml d'acétonitrile. On obtient après séchage à l'air puis en étuve héraeus (temp.40°C P: 0.2mbar) un deuxième lot de 6.59g de 4-(3-nitro-benzylamino)-1 H-pyrazole-3- carboxylique-acide-2.4-diméthoxy benzylamide sous la forme d'un solide jaune.
([M+H]+): 412). RT: 3.93 min (Méthode A)

Le 4-Amino-1H pyrazole-3-carboxylique acide-2.4- diméthoxy-benzylamide est préparé de la façon suivante :

A une suspension de 10.72g de 4-nitro-1H-pyrazole-3-carboxylique acid_2,4-diméthoxy-benzylamide dans 600ml d'éthanol absolu on ajoute par petites portions 27.64g de chlorure d'étain dihydrate. Le milieu réactionnel est agité 48 heures à 20°C. La solution limpide marron obtenue est amenée à sec à l'évaporateur rotatif La meringue marron claire obtenue est reprise avec700ml d'un mélange 90/10 en volumes de chlorure de méthylène / méthanol. A la solution brune obtenue on ajoute 700ml d'une solution aqueuse saturée en hydrogénocarbonate de sodium. La suspension crème obtenue est agitée 1 heure à température ambiante. On ajoute 30g de Clarcel Flo à la suspension puis on laisse agiter 10 minutes à température ambiante. On filtre, le gâteau est lavé avec 2 fois 250ml de mélange 90/10 en volumes de chlorure de méthylène / méthanol. Le solide est essoré, on récupère le filtrat puis on le transvase en ampoule à décanter. On décante la phase chlorométhylénique puis on extrait à nouveau la phase aqueuse avec 2 fois 250ml de dichlorométhane. Les phases organiques sont réunies puis séchées sur sulfate de magnésium, filtrées sur papier puis amenées à sec à l'évaporateur rotatif. On obtient 8.53g de 4-amino-1H pyrazole-3-carboxylique acide-2.4-diméthoxy-benzylamide sous la forme d'un solide rose pâle. ([M+H]+):277). RT: 2.36 min (Méthode A)

Le 4-nitro-1H-pyrazole-3-carboxylic acid 2,4-diméthoxy-benzylamide est obtenu de la façon suivante :

A une solution de 28.76g de 1-(3-diméthylaminopropyl)-3-éthyl carbodiimide chlorhydrate, et 20.27g de 1-hydroxybenzotriazole dans 100ml de diméthylformamide on ajoute 23g de 2,4-diméthoxy benzylamine, puis 20.04g de 4-nitro-3-pyrazole carboxylique acide à 98%. Le milieu réactionnel est agité à température ambiante pendant 20 heures. La solution jaune limpide obtenue est alors coulée sur 1000ml eau distillée. On obtient une suspension blanche qui est abandonnée 1 heure à température ambiante. On filtre la suspension, on lave le gâteau avec 3 fois 250ml eau distillée. Le solide obtenu est essoré, séché à l'air puis en étuve Héraeus sous vide ( temp. 40°C P : 0.2mbar).On obtient 40.65g d'un solide blanc, qui est trituré dans 750ml d'isopropanol au reflux pendant 20 minutes. La suspension obtenue est refroidie dans un bain eau +glace pendant 2 heures, puis filtrée. Le gâteau est lavé avec 2 fois 100ml d'isopropanol puis 2 fois 100ml d'éther isopropylique. Le solide obtenu est séché à l'air puis en étuve Héraeus (temp.40°C P: 0.2mbar). On obtient 32.16g de 4-Nitro-1H-pyrazole-3-carboxylic acid 2,4-diméthoxy-benzylamide sous la forme d'un solide blanc fondant à 204°C. ([M+H]+):307). RT: 3.12 min

### Exemple 35 : 4-(Éthyle-{3-[3-(2-fluoro-5-trifluorométhyl-phényl)-uréido]-benzyl}-amino)-1H-pyrazole-3-carboxamide

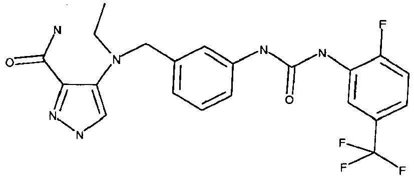

Le 4-(Éthyle-{3-[3-(2-fluoro-5-trifluorométhyl-phényl)-uréido]-benzyl}-amino)-1H-pyrazole-3-carboxamide est préparé par condensation du 1-fluoro-2-isocyanato-4-trifluorométhyl-benzène sur le 4-[(3-amino-benzyl)-éthyl-amino]-1 H-pyrazole-3-carboxamide selon le mode opératoire décrit à l'exemple 34, ce dernier dérivé étant lui même préparé à partir du 4-[(3-nitro-benzyl)-éthyl-amino]-1H-pyrazole-3-carboxamide selon le mode opératoire décrit également à l'exemple 34.

Le 4-[(3-nitro-benzyl)-éthyl-amino]-1H-pyrazole-3-carboxamide est obtenu de la façon suivante :

Une solution de 2.2g de chlorhydrate de 4-amino-1H pyrazole-3-carboxylique acide-2.4-diméthoxy-benzylamide, 1.3ml de diisopropyléthylamine dans 70 ml de THFest agitée pendant cinq minutes, puis on ajoute à cette solution 1.2g de 3-nitrobenzaldéhyde et 0.85 g de sulfate de magnésium. Le milieu réactionnel est porté au reflux pendant une heure, puis après refroidissement à 45°C on ajoute par petites portions 7.4g de triacétoxy borohydrure de sodium, puis on chauffe à nouveau au reflux pendant trois heures. Comme tout le produit de départ n'a pas disparu, après refroidissement à 45°C on ajoute par petites portions 7.4g de triacétoxy borohydrure de sodium, puis on chauffe à nouveau au reflux pendant deux heures. Après refroidissement à température ambiante du milieu réactionnel, on le coule sur 350ml d'eau distillée. La solution laiteuse jaune pâle ainsi obtenue est amenée à pH8-9 par ajout de 60 ml d'une solution aqueuse de soude 2N. On ajoute 250ml de dichlorométhane, puis après décantation, puis extraction de la phase aqueuse avec deux fois 150ml de dichlorométhane, on réunit les phases organiques, on lave avec 250ml de solution aqueuse saturée de chlorure de sodium, on les sèche sur sulfate de magnésium, filtre et concentre à sec sous pression réduite. On obtient 3.19g d'une huile collante qui est chromatographiée sur colonne de silice (90g de silice cartouche Merck de granulométrie de 15 à 45µm, diamètre de colonne 4.7 cm, fractions de 15ml, débit de 18ml/min, éluant acétate d'éthyle 70 cyclohexane 30 -en volumes). Les fractions 34 à 110 sont réunies, évaporées à sec sous pression réduite. On obtient 2.22g de 4-[(3-nitro-benzyl)-éthyl-amino]-1H-pyrazole-3-carboxamide sous la forme d'une meringue jaune. ([M+H]+): 453). RT:4.02 min

### (Méthode A)

### Exemple 36 : 4-({3-[3-(2-chloro-5-trifluorométhyl-phényl)-uréido]-benzyl}-méthyl-amino)-1H-pyrazole-3-carboxamide

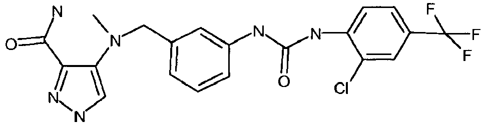

Le 4-({3-[3-(2-chloro-5-trifluorométhyl-phényl)-uréido]-benzyl}-méthyl-amino)-1H-pyrazole-3-carboxamide est obtenu par condensation du 1-chloro-2-isocyanato-4-trifluorométhyl-benzène sur le 4-[(3-amino-benzyl)-méthyl-amino]-1H-pyrazole-3-carboxamide selon le mode opératoire décrit à l'exemple 34.
([M+H]+): 467). RT: 3.81 min (Méthode A)

### Exemple 37: 4-{3-[3-(4-Trifluoromethyl-pyridin-2-yl)-ureido]-benzylamino}-1H-pyrazole-3-carboxamide

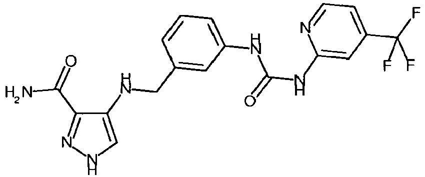

A une solution de 0.12g de 4-(3-amino-benzylamino)-1H-pyrazole-3-carboxamide dans 5ml de THF anhydre on ajoute à 20°C 88µl de triéthylamine et 0.18g de (4-trifluorométhyl-pyridin-2-yl) carbamic acide phényl ester. Le milieu réactionnel est chauffé 20 mn au microonde, puis, après refroidissement, dilué par 25ml d'acétate d'éthyle. La phase organique est lavée par deux fois 15ml d'eau distillée, puis décantée, séchée sur sulfate de magnésium et évaporée à sec sous pression réduite. Le résidu obtenu est chromatographié sur colonne de silice (15g de silice cartouche Merck de granulométrie de 15 à 45µm, diamètre de colonne 2.2 cm, fractions de 3.5ml, débit de 7ml/min, éluant acétate d'éthyle). Les fractions 36 à 60 sont réunies, évaporées à sec sous pression réduite. On obtient 0.06g de 4-{3-[3-(4-trifluorométhyl-pyridin-2-yl)-ureido]-benzylamino}-1H-pyrazole-3-carboxamide sous la forme d'un solide blanc. ([M+H]+): 420). RT: 0,78 min (Méthode D).

Le (4-trifluorométhyl-pyridin-2-yl) carbamic acide phényl ester est préparé de la façon suivante :

A une solution de 2-amino-4-trifluorométhyl-pyridine dans 65 ml de tétrahydrofurane anhydre on ajoute 0.81ml de pyridine, on refroidit le mélange réactionnel à 5°C puis on ajoute à cette température 0.95ml de phénylchloroformiate. Après agitation 2h à 5°C, on laisse revenir le mélange réactionnel à température ambiante, puis on le coule sur 20ml d'eau distillée en maintenant la température à 20°C. On ajoute ensuite 50ml d'acétate d'éthyle, on décante, la phase aqueuse est extraite deux fois par 20ml d'acétate d'éthyle. Les phases organiques sont réunies, séchées sur sulfate de Magnésium , filtrées puis évaporées à sec. Le solide obtenu est trituré dans 10ml de diisopropyl éther. On obtient 1.07g de 4-trifluorométhyl-pyridin-2-yl) carbamic acide phényl ester sous la forme d'un solide blanc fondant à 161°C. ([M+H]+): 281). RT:4,30min (Méthode A)

### Exemple 38 : 4-{3-[3-(4-méthoxy-pyridin-2-yl)-ureido]-benzylamino}-1H-pyrazole-3-carboxamide

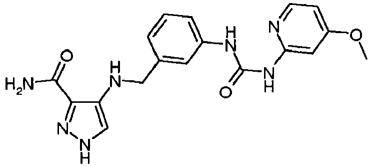

### Exemple 39 : (RS)-4-(1-{3-[3-(2-Chloro-4-trifluorométhyl-phényl)-ureido]-phényl}-éthylamino)-1 H-pyrazole-3-carboxamide

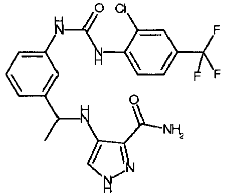

Les exemples 40 à 58, décrits dans le tableau A ci-dessous, sont obtenus ou pourraient être obtenus par condensation des isocyanates correspondants sur le 4-[(3-amino-benzyl)-amino]-1 H-pyrazole-3-carboxamide selon le mode opératoire décrit à l'exemple 34.

**Tableau A**

| Exemple N° | Structure | [M+H]+ (Méthode D) | RT (min) |
|---|---|---|---|
| 40 | 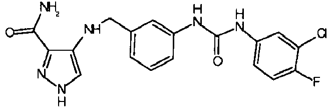 | 403 | 0,78 |
| 41 | 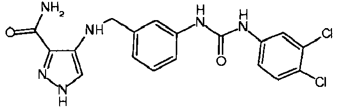 | 419 | 0,91 |
| 42 | 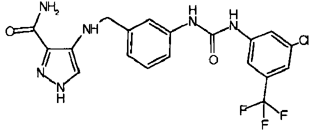 | 453 | 0,96 |
| 43 | 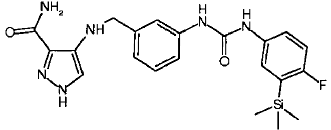 | 442 | n.d. |
| 44 | 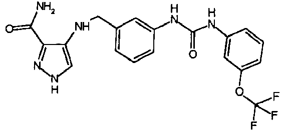 | 435 | n.d. |
| 45 | 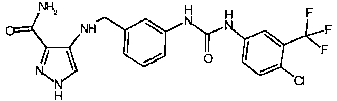 | 453 | 0,92 |
| 46 | 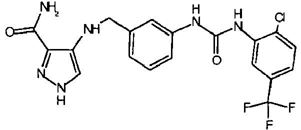 | 454 | n.d. |
| 47 | 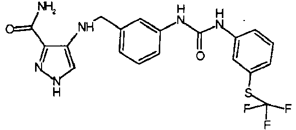 | 451 | 0,91 |
| 48 | 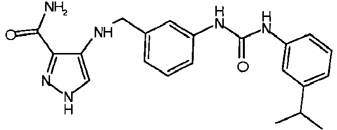 | 393 | 0,86 |
| 49 | 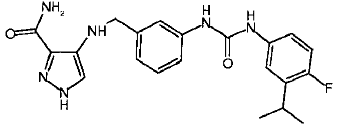 | 411 | n.d. |
| 50 | 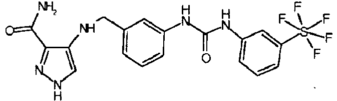 | 477 | n.d. |
| 51 | 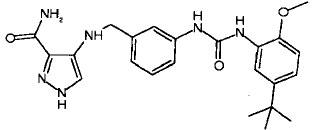 | 437 | 0,96 |
| 52 | 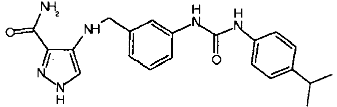 | 393 | 0,86 |
| 53 | 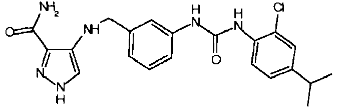 | 428 | n.d. |
| 54 | 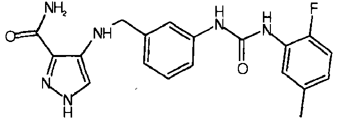 | 383 | 0,74 |
| 55 | 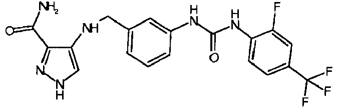 | 437 | 0,89 |
| 56 | 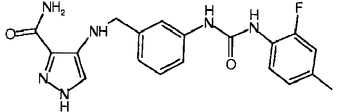 | 383 | 0,74 |
| 57 | 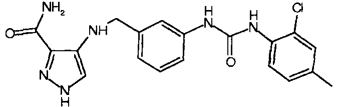 | 399 | 0,8 |
| 58 | 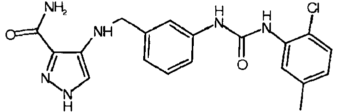 | 399 | 0,81 |

| | | | |
|---|---|---|---|
| n.d. non determiné | | | |

### Détermination de l'activité des composés - Protocoles expérimentaux

### 1. KDR

L'effet inhibiteur des composés est déterminé dans un test de phosphorylation de substrat par l'enzyme KDR *in vitro* par une technique de scintillation (plaque 96 puits, NEN).

Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLCγ exprimée sous forme de protéine de fusion GST), 2 µCi γ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).

Le bruit de fond est déterminé par la mesure de la radioactivité dans quatre puits différents contenant l'ATP radioactif et le substrat seul.

Un contrôle d'activité totale est mesuré dans quatre puits différents contenant tous les réactifs (γ³³P-[ATP], KDR et substrat PLCγ) mais en l'absence de composé.

L'inhibition de l'activité KDR avec le composé de l'invention est exprimée en pourcentage d'inhibition de l'activité contrôle déterminée en l'absence de composé.

Le composé SU5614 (Calbiochem) (1 µM) est inclus dans chaque plaque comme contrôle d'inhibition.

### 2. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80% d'homogénéité. Le substrat est composé des fragments SH2-SH3 de la PLC exprimée sous forme de protéine de fusion GST.

L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µL de tampon kinase contenant 100 ng d'enzyme GST-Tie2 par puits. Ensuite 10 µL de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi δ³³P[ATP]. Après 1 heure d'incubation à 37°C, la réaction est stoppée en ajoutant 1 volume (100µl) d'EDTA à 200 mM. Après élimination du tampon d'incubation, les puits sont lavés trois fois avec 300 µL de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé.

### Résultats :

Les composés des exemples de l'invention présente une concentration inhibant 50 % de l'activité de la kinase qui est généralement comprise entre 0.1 nM et 2 µM sur KDR et/ou TIE2, de préférence comprise entre 0.1 nM et 500 nM, et plus préférentiellement comprise entre 0.1 nM et 50 nM. Les valeurs du tableau 1, ci-dessous, sont données à titre d'illustration.

**Tableau 1 :**

| **Exemple** | **KDR** | **TIE2** |
|---|---|---|
| **2a** | 8 | 20 |
| **7** | 4,5 | 72,7 |
| **9** | 21,8 | 4510,4 |
| **13** | 11,8 | 1461,9 |
| **32** | 64,1 | 1000,8 |

## Revendications

1. Produit de formule générale (I) suivante : 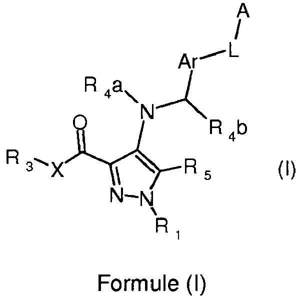 dans laquelle :
1) A et Ar sont indépendamment sélectionnés dans le groupe constitué par : aryle, hétéroaryle, aryle substitué, hétéroaryle substitué ;
1) L est sélectionné dans le groupe constitué par: NH-CO-NH et O-CO-NH ;
2) R₁ est sélectionné dans le groupe constitué par : H, R₆, COR₆, SO₂R₆, dans lequel R₆ est choisi parmi H, OR₇, NR₈R₉, alkyle, cycloalkyle, hétérocyclyle, hétérocyclyle substitué, aryle, aryle substitué, hétéroaryle, hétéroaryle substitué, dans lequel R₇ est choisi parmi H, phényle, alkyle, et dans lequel R₈ et R₉ sont indépendamment sélectionnés dans le groupe constitué par H, alkyle, cycloalkyle, hétérocyclyle, hétérocyclyle substitué, aryle, aryle substitué, hétéroaryle, hétéroaryle substitué ou bien R₈ et R₉ sont liés entre eux pour former un cycle saturé de 5 à 8 chaînons contenant de 0 à 3 hétéroatomes choisis parmi O, S et N ;
3) X est sélectionné dans le groupe constitué par : O et NH ;
4) R₃ est sélectionné dans le groupe constitué par : H, alkyle, alkyle substitué, cycloalkyle , cycloalkyle substitué ;
5) R₄ₐ est sélectionné dans le groupe constitué par : H ou (C1-C4)alkyle ;
6) R_{4b} est sélectionné dans le groupe constitué par : H ou (C1-C4)alkyle ;
7) R₅ est sélectionné dans le groupe constitué par : H, halogène, R₁₀, CN, O(R₁₀), OC(O)(R₁₀), OC(O)N(R₁₀)(R₁₁), OS(O₂)(R₁₀), N(R₁₀)(R₁₁), N=C(R₁₀)(R₁₁), N(R₁₀)C(O)(R₁₁), N(R₁₀)C(O)O(R₁₁), N(R₁₂)C(O)N(R₁₀)(R₁₁), N(R₁₂)C(S)N(R₁₀)(R₁₁), N(R₁₀)S(O₂)(R₁₁), C(O)(R₁₀), C(O)O(R₁₀), C(O)N(R₁₀)(R₁₁), C(=N(R₁₁))(R₁₀), C(=N(OR₁₁))(R₁₀), S(R₁₀), S(O)(R₁₀), S(O₂)(R₁₀), S(O₂)O(R₁₀), S(O₂)N(R₁₀)(R₁₁) ; dans lequel chaque R₁₀, R₁₁, R₁₂ est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué,
le terme " substitué " faisant référence à un ou plusieurs substituants différents de H, choisi parmi halogène; alkyle; aryle; hétéroaryle, cycloalkyle ; hétérocyclyle ; alkylène; alkynyle; OH ; O-alkyle; O-alkylène ; O-aryle; O-hétéroaryle; NH₂ ; NH-alkyle; NH-aryle; NH-hétéroaryle; N-alkyle-alkyle' où alkyle' et alkyle sont deux alkyles identiques ou différents ; SH ; S-alkyle; S-aryle; S(O₂)H; S(O₂)-alkyle; S(O₂)-aryle; SO₃H ; SO₃-alkyle; SO₃-aryle; CHO ; C(O)-alkyle; C(O)-aryle ; C(O)OH ; C(O)O-alkyle; C(O)O-aryle ; OC(O)- alkyle; OC(O)-aryle ; C(O)NH₂ ; C(O)NH-alkyle; C(O)NH-aryle ; NHCHO ; NHC(O)-alkyle; NHC(O)-aryle ; NH-cycloalkyle ; NH-hétérocyclyle.

2. Produit selon la revendication 1, **caractérisé en ce que** R₄ₐ et R_{4b} sont H.

3. Produit selon la revendication 1, **caractérisé en ce que** R₄ₐ est H et R_{4b} est (C1-C4)alkyle.

4. Produit selon la revendication 1, **caractérisé en ce que** R₄ₐ est (C1-C4)alkyle et R_{4b} est H.

5. Produit selon l'une quelconque des revendications 1 à 4, **caractérisé en ce que** R₁ est H

6. Produit selon l'une quelconque des revendications 1 à 5, **caractérisé en ce que** R₃ est H et X est NH.

7. Produit selon l'une quelconque des revendications 1 à 5, **caractérisé en ce que** R₃ est méthyle et X est O.

8. Produit selon l'une quelconque des revendications 1 à 7, **caractérisé en ce que** R₅ est H.

9. Produit selon l'une quelconque des revendications 1 à 8, **caractérisé en ce que** L est NHCONH.

10. Produit selon l'une quelconque des revendications 1 à 9, **caractérisé en ce que** Ar-L-A est : 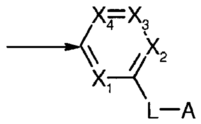 dans lequel chaque X₁, X₂, X₃ et X₄ est indépendamment choisi parmi N et C-R'₅, dans lequel R'₅ a la même définition que R₅.

11. Produit selon la revendication 10, **caractérisé en ce que** R'₅ est sélectionné dans le groupe constitué par H, F, Cl, méthyle, NH₂, OCF₃, et CONH₂.

12. Produit selon la revendication 9, **caractérisé en ce que** A est choisi parmi phényle, pyrazolyle et isoxazolyle ; éventuellement substitué.

13. Produit selon l'une quelconque des revendications 1 à 12, **caractérisé en ce que** A est substitué par un ou plusieurs substituants sélectionnés dans le groupe constitué par : H, F, Cl, Br, I, OH, SH, SO₃M, COOM, COO-alkyle, CON(R₁₄)(R₁₅), CN, NO_{2,} N(R₁₄)CO(R₁₅), N(R₁₄)(R₁₅), alkyle, alkyle halogéné, alkyle-OH, alkyle-N(R₁₄)(R₁₅), alkyle-(R₁₆), alkyle-COOM, alkyle-SO₃M, cycloalkyle, alkylène, alkynyle, aryle, hétéroaryle, O-alkyle, O-aryle, O-hétéroaryle, S-alkyle, S-aryle et S-hétéroaryle, chacun étant éventuellement substitué par un substituant choisi parmi alkyle, halogène, O-alkyle, N(R₁₄)(R₁₅) ; dans lequel R₁₄ et R₁₅ sont indépendamment choisis parmi H, alkyle, alkyle-OH, alkyle halogéné, alkyle-NH₂, alkyle-COOM, alkyle-SO₃M ; dans lequel lorsque R₁₄ et R₁₅ sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R₁₆ est H ou un hétérocycle non aromatique éventuellement substitué, comprenant 2 à 7 atomes de carbone, et 1 à 3 hétéroatomes choisis parmi N, O et S ; lorsque A est disubstitué, les deux substituants peuvent être liés entre eux pour former un cycle de 5 à 7 chaînons contenant de 0 à 3 hétéroatomes choisis parmi N, O et S.

14. Produit selon la revendication 13, **caractérisé en ce que** A est substitué par un ou plusieurs substituants sélectionnés dans ledit groupe complété par SiMe₃, S-CHF₃ et SF₅.

15. Produit selon l'une quelconque des revendications précédentes, **caractérisé en ce qu'**il s'agit de :
Trifluoroacétate de 4-{[3-phényl]carbamoyl}oxy)benzyl] amino}-1 H-pyrazole-3-carboxamide ;
Chlorhydrate de 4-{[3-({[2-fluoro-5-(trifluorométhyl) phényl]carbamoyl}amino)benzyl]amino}-1H-pyrazole-3-carboxamide ;
Trifluoroacétate de 4-[(3-{[(2-fluorophényl)carbamoyl]amino}benzyl)amino]-1H-pyrazole-3-carboxamide ;
Trifluoroacétate de 4-[(3-{[(2-méthoxyphényl) carbamoyl]amino}benzyl)amino]-1H-pyrazole-3-carboxamide ;
Trifluoroacétate de 4-{[3-({[2-fluoro-3-(trifluorométhyl) phényl]carbamoyl}amino)benzyl]amino}-1H-pyrazole-3-carboxamide ;
Trifluoroacétate de 4-[(3-{[(3-méthoxyphényl) carbamoyl]amino}benzyl)amino]-1H-pyrazole-3-carboxamide ;
Trifluoroacétate de 4-{[3-({[3-fluoro-5-(trifluorométhyl) phényl]carbamoyl}amino)benzyl]amino}-1H-pyrazole-3-carboxamide ;
Trifluoroacétate de 4-{[3-({[4-(trifluorométhoxy) phényl]carbamoyl}amino)benzyl]amino}-1 H-pyrazole-3-carboxamide ;
Trifluoroacétate de 3-{[(3-{[(3-carbamoyl-1H-pyrazol-4-yl)amino]méthyl}phényl)carbamoyl]amino}benzoate de méthyle ;
Trifluoroacétate de 4-{[3-({[4-(trifluorométhyl) phényl]carbamoyl}amino)benzyl]amino}-1H-pyrazole-3-carboxamide ;
Trifluoroacétate de 4-{[3-({[3-(trifluorométhyl) phényl]carbamoyl}amino)benzyl]amino}-1H-pyrazole-3-carboxamide ;
Trifluoroacétate de 4-{[3-({[2-(trifluorométhyl) phényl]carbamoyl}amino)benzyl]amino}-1H-pyrazole-3-carboxamide ;
Trifluoroacétate de 4-[(3-{[(3,5-diméthoxyphényl) carbamoyl]amino}benzyl)amino]-1H-pyrazole-3-carboxamide ;
Trifluoroacétate de 4-[(3-{[(3-méthylphényl)carbamoyl]amino}benzyl)amino]-1H-pyrazole-3-carboxamide ;
Trifluoroacétate de 4-[(3-{[(4-méthoxyphényl) carbamoyl]amino}benzyl)amino]-1H-pyrazole-3-carboxamide ;
Trifluoroacétate de 4-[(3-{[(4-fluorophényl) carbamoyl]amino}benzyl)amino]-1H-pyrazole-3-carboxamide ;
Trifluoroacétate de 4-{[3-({[4-chloro-3-(trifluorométhyl) phényl]carbamoyl}amino)benzyl]amino}-1H-pyrazole-3-carboxamide ;
Trifluoroacétate de 4-{[3-({[4-(difluorométhoxy)phényl]carbamoyl}amino)benzyl]amino}-1H-pyrazole-3-carboxamide ;
Trifluoroacétate de 4-{[3-({[2-chloro-4-(trifluorométhyl)phényl]carbamoyl}amino)benzyl]amino}-1H-pyrazole-3-carboxamide ;
Trifluoroacétate de 4-[(3-{[(4-méthylphényl) carbamoyl]amino}benzyl)amino]-1H-pyrazole-3-carboxamide ;
Trifluoroacétate de 4-[(3-{[(2,5-diméthylphényl) carbamoyl]amino}benzyl)amino]-1H-pyrazole-3-carboxamide ;
Trifluoroacétate de 4-[(3-{[(3,4-diméthylphényl)carbamoyl]amino}benzyl)amino]-1H-pyrazole-3-carboxamide ;
Trifluoroacétate de 4-[(3-{[(2-méthylphényl)carbamoyl]amino}benzyl)amino]-1H-pyrazole-3-carboxamide ;
Trifluoroacétate de 4-[(3-{[(3-éthylphényl)carbamoyl]amino}benzyl)amino]-1H-pyrazole-3-carboxamide ;
Trifluoroacétate de 4-{[3-({[3,5-bis(trifluorométhyl)phényl]carbamoyl}amino)benzyl]amino}-1H-pyrazole-3-carboxamide ;
Trifluoroacétate de 4-[(3-{[(3-fluorophényl) carbamoyl]amino}benzyl)amino]-1H-pyrazole-3-carboxamide ;
Trifluoroacétate de 4-[(3-{[(2-méthoxy-5-méthylphényl)carbamoyl]amino}benzyl)amino]-1H-pyrazole-3-carboxamide ;
Trifluoroacétate de 4-[(3-{[(2,5-diméthoxyphényl)carbamoyl]amino}benzyl)amino]-1H-pyrazole-3-carboxamide ;
Trifluoroacétate de 4-{[3-({[3-chloro-4-(difluorométhoxyphényl]carbamoyl}amino)benzyl]amino}-1H-pyrazole-3-carboxamide ;
Trifluoroacétate de 4-[(3-{[(2,5-difluorophényl) carbamoyl]amino}benzyl)amino]-1H-pyrazole-3-carboxamide ;
Trifluoroacétate de 4-{[3-({[4-méthyl-3-(trifluorométhyl)phényl]carbamoyl}amino)benzyl]amino}-1H-pyrazole-3-carboxamide;
Trifluoroacétate de 4-{[3-({[2-fluoro-5-(trifluorométhyl)phényl]carbamoyl}amino)benzyl]amino}-1H-pyrazole-3-carboxylate de méthyle.

16. Produit selon l'une quelconque des revendications précédentes, **caractérisé en ce qu'**il s'agit de :
Trifluoroacétate de 4-(1-{3-[3-(2-fluoro-5-trifluorométhyl-phényl)uréido]-phényl-éthylamino}-1H-pyrazole-3-carboxamide ;
4-({3-[3-(2-fluoro-5-trifluorométhyl-phényl)-uréido]-benzyl}-méthyl-amino)-1h-pyrazole-3-carboxamide ;
4-((Éthyle-{3-[3-(2-fluoro-5-trifluorométhyl-phényl)-uréido]-benzyl}-amino)-1H-pyrazole-3-carboxamide ;
4-({3-[3-(2-chloro-5-trifluorométhyl-phényl)-uréido]-benzyl}-méthyl-amino)-1H-pyrazole-3-carboxamide ;
4-{3-[3-(4-Trifluoromethyl-pyridin-2-yl)-ureido]-benzylamino}-1*H*-pyrazole-3-carboxamide ;
4-{3-[3-(4-méthoxy-pyridin-2-yl)-ureido]-benzylamino}-1*H*-pyrazole-3-carboxamide;
4-{[3-({[3-chloro-4-fluoro-phényl]carbamoyl}amino)benzyl]amino}-1H-pyrazole-3-carboxamide ;
4-{[3-({[3,4-dichloro-phényl]carbamoyl}amino)benzyl]amino}-1H-pyrazole-3-carboxamide ;
4-{[3-({[3-chloro-5-trifluorométhyl-phényl]carbamoyl}amino)benzyl]amino}-1H-pyrazole-3-carboxamide ;
4-{[3-({[3-triméthylsilyl-4-fluoro-phényl]carbamoyl}amino)benzyl]amino}-1H-pyrazole-3-carboxamide ;
4-{[3-({[3-trifluorométhoxy-phényl]carbamoyl}amino)benzyl]amino}-1H-pyrazole-3-carboxamide ;
4-{[3-({[3-trifluorométhyl-4-chloro-phényl]carbamoyl}amino)benzyl]amino}-1H-pyrazole-3-carboxamide ;
4-{[3-({[2-chloro-5-trifluorométhyl-phényl]carbamoyl}amino)benzyl]amino}-1H-pyrazole-3-carboxamide ;
4-{[3-({[3-trifluorométhylsulfanyl-phényl]carbamoyl}amino)benzyl]amino}-1H-pyrazole-3-carboxamide ;
4-{[3-({[3-isopropyl-phényl]carbamoyl}amino)benzyl]amino}-1H-pyrazole-3-carboxamide ;
4-{[3-({[3-isopropyl-4-fluoro-phényl]carbamoyl}amino)benzyl]amino}-1H-pyrazole-3-carboxamide ;
4-{[3-({[3-pentafluorosulfanyl-phényl]carbamoyl}amino)benzyl]amino}-1H-pyrazole-3-carboxamide ;
4-{[3-({[2-méthoxy-5-tertiobutyl-phényl]carbamoyl}amino)benzyl]amino}-1H-pyrazole-3-carboxamide ;
4-{[3-({[4-isopropyl-phényl]carbamoyl}amino)benzyl]amino}-1H-pyrazole-3-carboxamide ;
4-{[3-({[2-chloro-4-isopropyl-phényl]carbamoyl}amino)benzyl]amino}-1H-pyrazole-3-carboxamide ;
4-{[3-({[2-fluoro-5-méthyl-phényl]carbamoyl}amino)benzyl]amino}-1H-pyrazole-3-carboxamide ;
4-{[3-({[2-fluoro-4-trifluorométhyl-phényl]carbamoyl}amino)benzyl]amino}-1H-pyrazole-3-carboxamide ;
4-{[3-({[2-fluoro-4-méthyl-phényl]carbamoyl}amino)benzyl]amino}-1H-pyrazole-3-carboxamide ;
4-{[3-({[2-chloro-4-méthyl-phényl]carbamoyl}amino)benzyl]amino}-1H-pyrazole-3-carboxamide ;
4-{[3-({[2-chloro-5-méthyl-phényl]carbamoyl}amino)benzyl]amino}-1H-pyrazole-3-carboxamide ;
(RS)-4-(1-{3-[3-(2-Chloro-4-trifluorométhyl-phényl)-ureido]-phényl}-éthylamino)-1H-pyrazole-3-carboxamide.

17. Produit selon l'une quelconque des revendications précédentes, **caractérisé en ce qu'**il est sous forme :
1) non chirale, ou
2) racémique, ou
3) enrichie en un stéréo-isomère, ou
4) enrichie en un énantiomère ;
et **en ce qu'**il est éventuellement salifié.

18. Composition pharmaceutique comprenant un produit selon l'une quelconque des revendications 1 à 17, en combinaison avec un excipient pharmaceutiquement acceptable.

19. Utilisation d'un produit selon l'une quelconque des revendications 1 à 17, pour la fabrication d'un médicament utile pour traiter un état pathologique.

20. Utilisation selon la revendication 19, **caractérisée en ce que** l'état pathologique est le cancer.

21. Procédé de préparation des produits de formule générale (Ib) suivante : 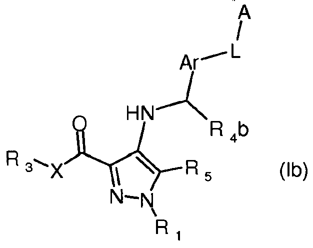 dans laquelle R₁, R₃, R_{4b}, R₅, X, Ar, L et A sont tels que définis à la revendication 1, et R₄ₐ est H, **caractérisé en ce qu'**un produit de formule générale (II) suivante : 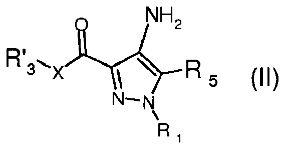 dans laquelle R'₃ est R₃ ou un précurseur de R₃, et X, R₁, R₃ et R₅ sont tels que définis à la revendication 1, réagit avec un produit de formule (III) suivante : 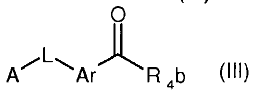 dans laquelle R_{4b}, Ar, L et A sont tels que définis à la revendication 1, pour obtenir le produit de formule générale (Ib).

22. Procédé de préparation des produits de formule générale (I) suivante : 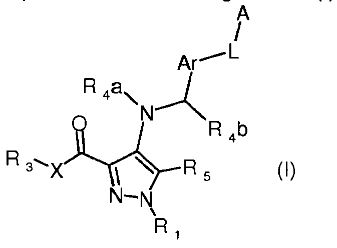 dans laquelle R₁, R₃, R₄ₐ, R_{4b}, R₅, X, Ar et A sont tels que définis à la revendication 1, et L est NHCONH, **caractérisé en ce qu'**un produit de formule générale (VIII) suivante : 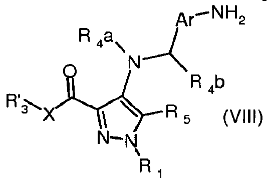 dans laquelle R'₃ est R₃ ou un précurseur de R₃, et X, Ar, R₁, R₃, R₄ₐ, R_{4b} et R₅ sont tels que définis à la revendication 1, réagit avec un produit de formule (VII) suivante : 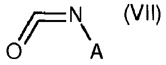 dans laquelle A est tel que défini à la revendication 1, pour obtenir le produit de formule générale (I') de formule suivante 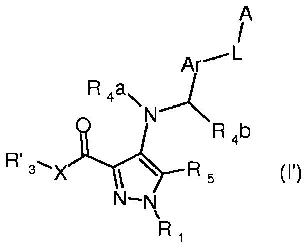 où le précurseur R'₃ est transformé en R₃ afin d'obtenir le produit de formule générale (I).

23. A titre de produits intermédiaires, les produits de formule générale (VIII), pour lesquels Ar, R'₃, X, R₁, R₄ₐ, R_{4b} et R₅ sont tels que définis à la revendication 22.

## Patentansprüche

1. Produkt der folgenden allgemeinen Formel (I): 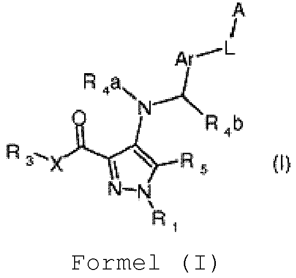 worin:
1) A und Ar unabhängig voneinander aus der Gruppe bestehend aus Aryl, Heteroaryl, substituiertem Aryl und substituiertem Heteroaryl ausgewählt sind;
1) L aus der Gruppe bestehend aus NH-CO-NH und O-CO-NH ausgewählt ist;
2) R₁ aus der Gruppe bestehend aus H, R₆, COR₆, SO₂R₆ ausgewählt ist, wobei R₆ aus H, OR₇, NR₈R₉, Alkyl, Cycloalkyl, Heterocyclyl, substituiertem Heterocyclyl, Aryl, substituiertem Aryl, Heteroaryl und substituiertem Heteroaryl ausgewählt ist, wobei R₇ aus H, Phenyl und Alkyl ausgewählt ist und wobei R₈ und R₉ unabhängig voneinander aus der Gruppe bestehend aus H, Alkyl, Cycloalkyl, Heterocyclyl, substituiertem Heterocyclyl, Aryl, substituiertem Aryl, Heteroaryl und substituiertem Heteroaryl ausgewählt sind oder R₈ und R₉ unter Bildung eines gesättigten 5- bis 8-gliedrigen Rings, der 0 bis 3 aus 0, S und N ausgewählte Heteroatome enthält, miteinander verbunden sind;
3) X aus der Gruppe bestehend aus 0 und NH ausgewählt ist;
4) R₃ aus der Gruppe bestehend aus H, Alkyl, substituiertem Alkyl, Cycloalkyl und substituiertem Cycloalkyl ausgewählt ist;
5) R₄ₐ aus der Gruppe bestehend aus H oder (C1-C4)-Alkyl ausgewählt ist;
6) R_{4b} aus der Gruppe bestehend aus H oder (C1-C4)-Alkyl ausgewählt ist;
7) R₅ aus der Gruppe bestehend aus H, Halogen, R₁₀, CN, O(R₁₀), OC(O)(R₁₀), OC(O)N(R₁₀) (R₁₁), OS(O₂) (R₁₀), N(R₁₀)(R₁₁), N=C(R₁₀)(R₁₁), N(R₁₀)C(O)(R₁₁), N(R₁₀)C(O)O(R₁₁), N(R₁₂)C(O)N(R₁₀)(R₁₁), N(R₁₂)C(S)N(R₁₀)(R₁₁), N(R₁₀)S(O₂)(R₁₁), C(O)(R₁₀), C(O)O(R₁₀), C(O)N(R₁₀) (R₁₁), C(=N(R₁₁))(R₁₀), C(=N(OR₁₁))(R₁₀), S(R₁₀), S(O)(R₁₀), S(O₂)(R₁₀), S(O₂)O(R₁₀) und S(O₂)N(R₁₀)(R₁₁) ausgewählt ist; wobei R₁₀, R₁₁ und R₁₂ jeweils unabhängig aus der Gruppe bestehend aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl und substituiertem Heterocyclyl ausgewählt sind,
wobei der Begriff "substituiert" sich auf einen oder mehrere von H verschiedene Substituenten bezieht, die aus Halogen; Alkyl; Aryl; Heteroaryl; Cycloalkyl; Heterocyclyl; Alkylen; Alkinyl; OH; 0-Alkyl; 0-Alkylen; 0-Aryl; 0-Heteroaryl; NH₂; NH-Alkyl; NH-Aryl; NH-Heteroaryl; N-Alkyl-alkyl', worin Alkyl' und Alkyl für zwei gleiche oder verschiedene Alkylgruppen stehen; SH; S-Alkyl; S-Aryl; S(O₂)H; S(O₂)-Alkyl; S(O₂)-Aryl; SO₃H; SO₃-Alkyl; SO₃-Aryl; CHO; C(O)-Alkyl; C(0)-Aryl; C(O)OH; C(O)O-Alkyl; C(O)O-Aryl; OC(O)-Alkyl; OC(O)-Aryl; C(O)NH₂; C(0)NH-Alkyl; C(O)NH-Aryl; NHCHO; NHC(0)-Alkyl; NHC(O)-Aryl; NH-Cycloalkyl und NH-Heterocyclyl ausgewählt sind.

2. Produkt nach Anspruch 1, **dadurch gekennzeichnet, dass** R₄ₐ und R_{4b} für H stehen.

3. Produkt nach Anspruch 1, **dadurch gekennzeichnet, dass** R₄ₐ für H steht und R_{4b} für (C1-C4)-Alkyl steht.

4. Produkt nach Anspruch 1, **dadurch gekennzeichnet, dass** R₄ₐ für (C1-C4)-Alkyl steht und R_{4b} für H steht.

5. Produkt nach einem der Ansprüche 1 bis 4, **dadurch gekennzeichnet, dass** R₁ für H steht.

6. Produkt nach einem der Ansprüche 1 bis 5, **dadurch gekennzeichnet, dass** R₃ für H steht und X für NH steht.

7. Produkt nach einem der Ansprüche 1 bis 5, **dadurch gekennzeichnet, dass** R₃ für Methyl steht und X für O steht.

8. Produkt nach einem der Ansprüche 1 bis 7, **dadurch gekennzeichnet, dass** R₅ für H steht.

9. Produkt nach einem der Ansprüche 1 bis 8, **dadurch gekennzeichnet, dass** L für NHCONH steht.

10. Produkt nach einem der Ansprüche 1 bis 9, **dadurch gekennzeichnet, dass** Ar-L-A für 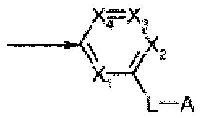 steht, wobei X₁, X₂, X₃, und X₄ jeweils unabhängig aus N und C-R'₅ ausgewählt sind, wobei R'₅ die gleiche Definition wie R₅ besitzt.

11. Produkt nach Anspruch 10, **dadurch gekennzeichnet, dass** R'₅ aus der Gruppe bestehend aus H, F, Cl, Methyl, NH₂, OCF₃ und CONH₂ ausgewählt ist.

12. Produkt nach Anspruch 9, **dadurch gekennzeichnet, dass** A aus gegebenenfalls substituiertem Phenyl, Pyrazolyl und Isoxazolyl ausgewählt ist.

13. Produkt nach einem der Ansprüche 1 bis 12, **dadurch gekennzeichnet, dass** A durch einen oder mehrere Substituenten substituiert ist, die aus der Gruppe bestehend aus H, F, Cl, Br, I, OH, SH, SO₃M, COOM, COO-Alkyl, CON(R₁₄)(R₁₅), CN, NO₂,N(R₁₄)CO(R₁₅), N(R₁₄)(R₁₅), Alkyl, Halogenalkyl, Alkyl-OH, Alkyl-N(R₁₄)(R₁₅), Alkyl (R₁₆), Alkyl-COOM, Alkyl-SO₃M, Cycloalkyl, Alkylen, Alkinyl, Aryl, Heteroaryl, 0-Alkyl, 0-Aryl, O-Heteroaryl, S-Alkyl, S-Aryl und S-Heteroaryl ausgewählt sind, wobei jeder dieser Substituenten gegebenenfalls durch einen aus Alkyl, Halogen, 0-Alkyl und N(R₁₄)(R₁₅) ausgewählten Substituenten substituiert ist; wobei R₁₄ und R₁₅ unabhängig voneinander aus H, Alkyl, Alkyl-OH, Halogenalkyl, Alkyl-NH₂, Alkyl-COOM und Alkyl-SO₃M ausgewählt sind; wobei dann, wenn R₁₄ und R₁₅ gleichzeitig von H verschieden sind, zur Bildung eines 5- bis 7-gliedrigen Rings, der 0 bis 3 aus 0, N und S ausgewählte Heteroatome enthält, verbunden sein können; wobei M für H oder ein Kation eines aus Li, Na und K ausgewählten Alkalimetalls steht; und wobei R₁₆ für H oder einen gegebenenfalls substituierten nicht aromatischen Heterocyclus mit 2 bis 7 Kohlenstoffatomen und 1 bis 3 aus N, 0 und S ausgewählten Heteroatomen steht; wobei dann, wenn A disubstituiert ist, die beiden Substituenten miteinander zur Bildung eines 5- bis 7-gliedrigen Rings, der 0 bis 3 aus N, 0 und S ausgewählten Heteroatome enthält, miteinander verbunden sein können, ausgewählt sind.

14. Produkt nach Anspruch 13, **dadurch gekennzeichnet, dass** A durch einen oder mehrere Substituenten substituiert ist, die aus der durch SiMe₃, S-CHF₃ und SF₅ komplettierten Gruppe ausgewählt sind.

15. Produkt nach einem der vorhergehenden Ansprüche, **dadurch gekennzeichnet, dass** es sich um
4-{[3-Phenyl]carbamoyl}oxy)benzyl]amino}-1H-pyrazol-3-carboxamid-trifluoracetat;
4-{[3-({[2-Fluor-5-(trifluormethyl)phenyl]-carbamoyl}amino)benzyl]amino}-1H-pyrazol-3-carboxamid-hydrochlorid;
4-[((3-{[(2-Fluorphenyl)carbamoyl]amino}benzyl)-amino]-1H-pyrazol-3-carboxamid-trifluoracetat;
4-[((3-{[(2-Methoxyphenyl)carbamoyl]amino}-benzyl)amino]-1H-pyrazol-3-carboxamid-trifluoracetat;
4-{[3-({[2-Fluor-3-(trifluormethyl)phenyl]-carbamoyl}amino)benzyl]amino}-1H-pyrazol-3-carboxamid-trifluoracetat;
4-[((3-{[(3-Methoxyphenyl)carbamoyl]amino}benzyl)-amino]-1H-pyrazol-3-carboxamid-trifluoracetat;
4-{[3-({[3-Fluor-5-(trifluormethyl)phenyl]-carbamoyl}amino)benzyl]amino}-1H-pyrazol-3-carboxamid-trifluoracetat;
4-{[3-({[4-(Trifluormethoxy)phenyl]carbamoyl}-amino)benzyl]amino}-1H-pyrazol-3-carboxamid-trifluoracetat;
3-{[((3-{[(3-Carbamoyl-1H-pyrazol-4-yl)amino]-methyl}phenyl)carbamoyl]amino}benzoesäuremethyl-ester-trifluoracetat;
4-{[3-({[4-(Trifluormethyl)phenyl]carbamoyl}-amino)benzyl]amino}-1H-pyrazol-3-carboxamid-trifluoracetat;
4-{[3-({[3-(Trifluormethyl)phenyl]carbamoyl}-amino)benzyl]amino}-1H-pyrazol-3-carboxamid-trifluoracetat;
4-{[3-({[2-(Trifluormethyl)phenyl]carbamoyl}-amino)benzyl]amino}-1H-pyrazol-3-carboxamid-trifluoracetat;
4-[((3-{[(3,5-Dimethoxyphenyl)carbamoyl]amino}-benzyl)amino]-1H-pyrazol-3-carboxamid-trifluoracetat;
4-[((3-{[(3-Methylphenyl)carbamoyl]amino}benzyl)-amino]-1H-pyrazol-3-carboxamid-trifluoracetat;
4-[((3-{[(4-Methoxyphenyl)carbamoyl]amino}benzyl)-amino]-1H-pyrazol-3-carboxamid-trifluoracetat;
4-[((3-{[(4-Fluorphenyl)carbamoyl]amino}benzyl)-amino]-1H-pyrazol-3-carboxamid-trifluoracetat;
4-{[3-({[4-Chlor-3-(trifluormethyl)phenyl]-carbamoyl}amino)benzyl]amino}-1H-pyrazol-3-carboxamid-trifluoracetat;
4-{[3-({[4-(Difluormethoxy)phenyl]carbamoyl}-amino)benzyl]amino}-1H-pyrazol-3-carboxamid-trifluoracetat;
4-{[3-({[2-Chlor-4-(trifluormethyl)phenyl]-carbamoyl}amino)benzyl]amino}-1H-pyrazol-3-carboxamid-trifluoracetat;
4-[((3-{[(4-Methylphenyl)carbamoyl]amino}benzyl)-amino]-1H-pyrazol-3-carboxamid-trifluoracetat;
4-[((3-{[(2,5-Dimethylphenyl)carbamoyl]amino}-benzyl)amino]-1H-pyrazol-3-carboxamid-trifluoracetat;
4-[((3-{[(3,4-Dimethylphenyl)carbamoyl]amino}-benzyl)amino]-1H-pyrazol-3-carboxamid-trifluoracetat;
4-[((3-{[(2-Methylphenyl)carbamoyl]amino}benzyl)-amino]-1H-pyrazol-3-carboxamid-trifluoracetat;
4-[((3-{[(3-Ethylphenyl)carbamoyl]amino}benzyl)-amino]-1H-pyrazol-3-carboxamid-trifluoracetat;
4-{[3-({[3,5-Bis(trifluormethyl)phenyl]carbamoyl}-amino)benzyl]amino}-1H-pyrazol-3-carboxamid-trifluoracetat;
4-[((3-{[(3-Fluorphenyl)carbamoyl]amino}benzyl)-amino]-1H-pyrazol-3-carboxamid-trifluoracetat;
4-[((3-{[(2-Methoxy-5-methylphenyl)carbamoyl]-amino}benzyl)amino]-1H-pyrazol-3-carboxamid-trifluoracetat;
4-[((3-{[(2,5-Dimethoxyphenyl)carbamoyl]amino}-benzyl)amino]-1H-pyrazol-3-carboxamid-trifluoracetat;
4-{[3-({[3-Chlor-4-(difluormethoxyphenyl]-carbamoyl}amino)benzyl]amino}-1H-pyrazol-3-carboxamid-trifluoracetat;
4-[((3-{[(2,5-Difluorphenyl)carbamoyl]amino}-benzyl)amino]-1H-pyrazol-3-carboxamid-trifluoracetat;
4-{[3-({[4-Methyl-3-(trifluormethyl)phenyl]-carbamoyl}amino)benzyl]amino}-1H-pyrazol-3-carboxamid-trifluoracetat;
4-{[3-({[2-Fluor-5-(trifluormethyl)phenyl]-carbamoyl}amino)benzyl]amino}-1H-pyrazol-3-carbonsäuremethylester-trifluoracetat handelt.

16. Produkt nach einem der vorhergehenden Ansprüche, **dadurch gekennzeichnet, dass** es sich um
4-((1-{3-[3-(2-Fluor-5-trifluormethylphenyl)-ureido]phenylethylamino}-1H-pyrazol-3-carboxamid-trifluoracetat;
4-({3-[3-(2-Fluor-5-trifluormethylphenyl)ureido]-benzyl}methylamino)-1H-pyrazol-3-carboxamid;
4-((Ethyl-{3-[3-(2-fluor-5-trifluormethylphenyl)-ureido]benzyl}amino)-1H-pyrazol-3-carboxamid;
4-({3-[3-(2-Chlor-5-trifluormethylphenyl)ureido]-benzyl}methylamino)-1H-pyrazol-3-carboxamid;
4-{3-[3-(4-Trifluormethylpyridin-2-yl)ureido]-benzylamino}-1H-pyrazol-3-carboxamid;
4-{3-[3-(4-Methoxypyridin-2-yl)ureido]benzylamino}-1H-pyrazol-3-carboxamid;
4-{[3-({[3-Chlor-4-fluorphenyl]carbamoyl}amino)-benzyl]amino}-1H-pyrazol-3-carboxamid;
4-{[3-({[3,4-Dichlorphenyl]carbamoyl}amino)-benzyl]amino}-1H-pyrazol-3-carboxamid;
4-{[3-({[3-Chlor-5-trifluormethylphenyl]-carbamoyl}amino)benzyl]amino}-1H-pyrazol-3-carboxamid;
4-{[3-({[3-Trimethylsilyl-4-fluorphenyl]-carbamoyl}amino)benzyl]amino}-1H-pyrazol-3-carboxamid;
4-{[3-({[3-Trifluormethoxyphenyl]carbamoyl}amino)-benzyl]amino}-1H-pyrazol-3-carboxamid;
4-{[3-({[3-Trifluormethyl-4-chlorphenyl]-carbamoyl}amino)benzyl]amino}-1H-pyrazol-3-carboxamid;
4-{[3-({[2-Chlor-5-trifluormethylphenyl]-carbamoyl}amino)benzyl]amino}-1H-pyrazol-3-carboxamid;
4-{[3-({[3-Trifluormethylsulfanylphenyl]-carbamoyl}amino)benzyl]amino}-1H-pyrazol-3-carboxamid;
4-{[3-({[3-Isopropylphenyl]carbamoyl}amino)-benzyl]amino}-1H-pyrazol-3-carboxamid;
4-{[3-({[3-Isopropyl-4-fluorphenyl]carbamoyl}-amino)benzyl]amino}-1H-pyrazol-3-carboxamid;
4-{[3-({[3-Pentafluorsulfanylphenyl]carbamoyl}-amino)benzyl]amino}-1H-pyrazol-3-carboxamid;
4-{[3-({[2-Methoxy-5-tert-butylphenyl]carbamoyl}-amino)benzyl]amino}-1H-pyrazol-3-carboxamid;
4-{[3-({[4-Isopropylphenyl]carbamoyl}amino)-benzyl]amino}-1H-pyrazol-3-carboxamid;
4-{[3-({[2-Chlor-4-isopropylphenyl]carbamoyl}-amino)benzyl]amino}-1H-pyrazol-3-carboxamid;
4-{[3-({[2-Fluor-5-methylphenyl]carbamoyl}amino)-benzyl]amino}-1H-pyrazol-3-carboxamid;
4-{[3-({[2-Fluor-4-trifluormethylphenyl]-carbamoyl}amino)benzyl]amino}-1H-pyrazol-3-carboxamid;
4-{[3-({[2-Fluor-4-methylphenyl]carbamoyl}amino)-benzyl]amino}-1H-pyrazol-3-carboxamid;
4-{[3-({[2-Chlor-4-methylphenyl]carbamoyl}amino)-benzyl]amino}-1H-pyrazol-3-carboxamid;
4-{[3-({[2-Chlor-5-methylphenyl]carbamoyl}amino)-benzyl]amino}-1H-pyrazol-3-carboxamid;
(RS)-4-(1-{3-[3-(2-Chlor-4-trifluormethylphenyl)-ureido]phenyl}ethylamino)-1H-pyrazol-3-carboxamid handelt.

17. Produkt nach einem der vorhergehenden Ansprüche, **dadurch gekennzeichnet, dass** es
a) in achiraler Form oder
b) in racemischer Form oder
c) in mit einem Stereoisomer angereicherter Form oder
d) in mit einem Enantiomer angereicherter Form vorliegt und gegebenenfalls versalzt ist.

18. Pharmazeutische Zusammensetzung, umfassend ein Produkt nach einem der Ansprüche 1 bis 17 in Kombination mit einem pharmazeutisch unbedenklichen Exzipienten.

19. Verwendung eines Produkts nach einem der Ansprüche 1 bis 17 zur Herstellung eines Medikaments zur Behandlung eines pathologischen Zustands.

20. Verwendung nach Anspruch 19, **dadurch gekennzeichnet, dass** es sich bei dem pathologischen Zustand um Krebs handelt.

21. Verfahren zur Herstellung der Produkte der folgenden allgemeinen Formel (Ib): 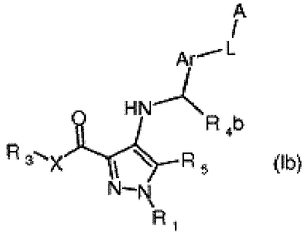 worin R₁, R₃, R_{4b}, R₅, X, Ar, L und A die in Anspruch 1 angegebene Bedeutung besitzen und R₄ₐ für H steht, **dadurch gekennzeichnet, dass** man ein Produkt der folgenden allgemeinen Formel (II): 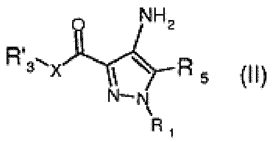 worin R'₃ für R₃ oder einen Vorläufer von R₃ steht und X, R₁, R₃ und R₅ die in Anspruch 1 angegebene Bedeutung besitzen, mit einem Produkt der folgenden Formel (III): 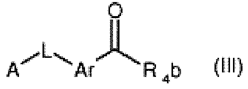 worin R_{4b}, Ar, L und A die in Anspruch 1 angegebene Bedeutung besitzen, zu dem Produkt der allgemeinen Formel (Ib) umsetzt.

22. Verfahren zur Herstellung der Produkte der folgenden allgemeinen Formel (I): 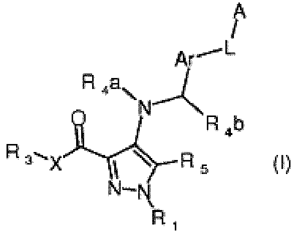 worin R₁, R₃, R₄ₐ, R_{4b}, R₅, X, Ar und A die in Anspruch 1 angegebene Bedeutung besitzen und L für NHCONH steht, **dadurch gekennzeichnet, dass** man ein Produkt der folgenden allgemeinen Formel (VIII): 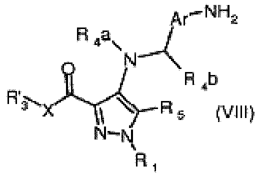 worin R'₃ für R₃ oder einen Vorläufer von R₃ steht und X, Ar, R₁, R₃, R₄ₐ, R_{4b} und R₅ die in Anspruch 1 angegebene Bedeutung besitzen, mit einem Produkt der folgenden Formel (VII): 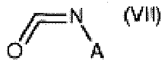 worin A die in Anspruch 1 angegebene Bedeutung besitzt, zu dem Produkt der allgemeinen Formel (I') der folgenden Formel 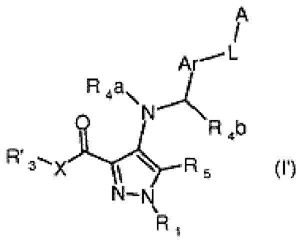 umsetzt, wobei der Vorläufer R'₃ in R₃ umgewandelt wird, wobei man das Produkt der allgemeinen Formel (I) erhält.

23. Als Zwischenprodukte die Produkte der allgemeinen Formel (VIII), für die Ar, R'₃, X, R₁, R₄ₐ, R_{4b} und R₅ die in Anspruch 22 angegebene Bedeutung besitzen.

## Claims

1. Product of general formula (I) below: 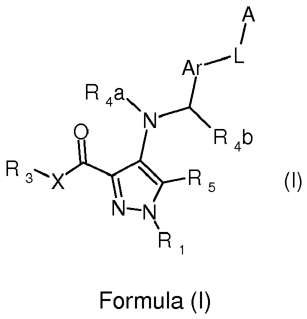 in which:
1) A and Ar are independently selected from the group consisting of: aryl, heteroaryl, substituted aryl, substituted heteroaryl;
2) L is selected from the group consisting of: NH-CO-NH and O-CO-NH;
3) R₁ is selected from the group consisting of: H, R₆, COR₆, SO₂R₆, in which R₆ is chosen from H, OR₇, NR₈R₉, alkyl, cycloalkyl, heterocyclyl, substituted heterocyclyl, aryl, substituted aryl, heteroaryl and substituted heteroaryl, in which R₇ is chosen from H, phenyl and alkyl, and in which R₈ and R₉ are independently selected from the group consisting of H, alkyl, cycloalkyl, heterocyclyl, substituted heterocyclyl, aryl, substituted aryl, heteroaryl and substituted heteroaryl, or alternatively R₈ and R₉ are linked together to form a saturated 5- to 8-membered ring containing from 0 to 3 heteroatoms chosen from O, S and N;
4) X is selected from the group consisting of: O and NH;
5) R₃ is selected from the group consisting of: H, alkyl, substituted alkyl, cycloalkyl, substituted cycloalkyl;
6) R₄ₐ is selected from the group consisting of: H and (C1-C4)alkyl;
7) R_{4b} is selected from the group consisting of: H and (C1-C4)alkyl;
8) R₅ is selected from the group consisting of: H, halogen, R₁₀, CN, O(R₁₀), OC(O)(R₁₀), OC(O)N(R₁₀)(R₁₁), OS(O₂)(R₁₀), N(R₁₀)(R₁₁), N=C(R₁₀)(R₁₁), N(R₁₀)C(O)(R₁₁), N(R₁₀)C(O)O(R₁₁), N(R₁₂)C(O)N(R₁₀)(R₁₁), N(R₁₂)C(S)N(R₁₀)(R₁₁), N(R₁₀)S(O₂)(R₁₁), C(O)(R₁₀), C(O)O(R₁₀), C(O)N(R₁₀)(R₁₁), C(=N(R₁₁))(R₁₀), C(=N(OR₁₁)(R₁₀), S(R₁₀), S(O)(R₁₀), S(O₂)(R₁₀), S(O₂)O(R₁₀), S(O₂)N(R₁₀)(R₁₁); in which each R₁₀, R₁₁, R₁₂ is independently selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl and substituted heterocyclyl.,
the term "substituted" referring to one or more substituents other than H, selected from halogen; alkyl; aryl; heteroaryl, cycloalkyl; heterocyclyl; alkylene; alkynyl; OH; O-alkyl; O-alkylene; O-aryl; O-heteroaryl; NH₂; NH-alkyl; NH-aryl; NH-heteroaryl; N-alkyl-alkyl', in which alkyl' and alkyl are two identical or different alkyls; SH; S-alkyl; S-aryl; S(O₂)H; S(O₂)-alkyl; S(O₂)-aryl; SO₃H; SO₃-alkyl; SO₃-aryl; CHO; C(O)-alkyl; C(O)-aryl; C(O)OH; C(O)O-alkyl; C(O)O-aryl; OC(O)-alkyl; OC(O)-aryl; C(O)NH₂; C(O)NH-alkyl; C(O)NH-aryl; NHCHO; NHC(O)-alkyl; NHC(O)-aryl; NH-cycloalkyl; and NH-heterocyclyl.

2. Product according to Claim 1, **characterized in that** R₄ₐ and R_{4b} are H.

3. Product according to Claim 1, **characterized in that** R₄ₐ is H and R_{4b} is (C1-C4)alkyl.

4. Product according to Claim 1, **characterized in that** R₄ₐ is (C1-C4)alkyl and R_{4b} is H.

5. Product according to any one of Claims 1 to 4, **characterized in that** R₁ is H.

6. Product according to any one of Claims 1 to 5, **characterized in that** R₃ is H and X is NH.

7. Product according to any one of Claims 1 to 5, **characterized in that** R₃ is methyl and XisO.

8. Product according to any one of Claims 1 to 7, **characterized in that** R₅ is H.

9. Product according to any one of Claims 1 to 8, **characterized in that** L is NHCONH.

10. Product according to any one of Claims 1 to 9, **characterized in that** Ar-L-A is: 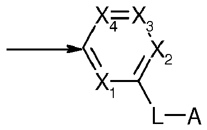 in which each X₁, X₂, X₃ and X₄ is independently chosen from N and C-R'₅, in which R'₅ has the same definition as R₅.

11. Product according to Claim 10, **characterized in that** R'₅ is selected from the group consisting of H, F, Cl, methyl, NH₂, OCF₃ and CONH₂.

12. Product according to Claim 9, **characterized in that** A is chosen from phenyl, pyrazolyl and isoxazolyl; optionally substituted.

13. Product according to any one of Claims 1 to 12, **characterized in that** A is substituted with one or more substituents selected from the group consisting of: H, F, Cl, Br, I, OH, SH, SO₃M, COOM, COO-alkyl, CON(R₁₄)(R₁₅), CN, NO₂, N(R₁₄)CO(R₁₅), N(R₁₄)(R₁₅), alkyl, haloalkyl, alkyl-OH, alkyl-N(R₁₄)(R₁₅), alkyl(R₁₆), alkyl-COOM, alkyl-SO₃M, cycloalkyl, alkylene, alkynyl, aryl, heteroaryl, O-alkyl, O-aryl, O-heteroaryl, S-alkyl, S-aryl and S-heteroaryl, each being optionally substituted with a substituent chosen from alkyl, halogen, O-alkyl and N(R₁₄)(R₁₅); in which R₁₄ and R₁₅ are independently chosen from H, alkyl, alkyl-OH, haloalkyl, alkyl-NH₂, alkyl-COOM and alkyl-SO₃M; in which, when R₁₄ and R₁₅ are simultaneously other than H, may be bonded to form a 5- to 7-membered ring comprising from 0 to 3 heteroatoms chosen from O, N and S; in which M is H or a cation of an alkali metal chosen from Li, Na and K; and in which R₁₆ is H or an optionally substituted non-aromatic heterocycle, containing from 2 to 7 carbon atoms, and 1 to 3 heteroatoms chosen from N, O and S; when A is disubstituted, the two substituents may be linked together to form a 5- to 7-membered ring containing from 0 to 3 heteroatoms chosen from N, O and S.

14. Product according to Claim 13, **characterized in that** A is substituted with one or more substituents selected from the said group, also including SiMe₃, S-CHF₃ and SF₅.

15. Product according to any one of the preceding claims, **characterized in that** it is:
4-{[3-phenyl]carbamoyl}oxy)benzyl]amino}-1H-pyrazole-3-carboxamide trifluoroacetate;
4-{[3-({[2-fluoro-5-(trifluoromethyl)phenyl]carbamoyl}amino)benzyl]amino}-1H-pyrazole-3-carboxamide hydrochloride;
4-[((3-{[(2-fluorophenyl)carbamoyl]amino}benzyl)amino]-1H-pyrazole-3-carboxamide trifluoroacetate;
4-[((3-{[(2-methoxyphenyl)carbamoyl]amino}benzyl)amino]-1H-pyrazole-3-carboxamide trifluoroacetate;
4-{[3-({[2-fluoro-3-(trifluoromethyl)phenyl]carbamoyl}amino)benzyl]amino}-1H-pyrazole-3-carboxamide trifluoroacetate;
4-[((3-{[(3-methoxyphenyl)carbamoyl]amino}benzyl)amino]-1H-pyrazole-3-carboxamide trifluoroacetate;
4-{[3-({[3-fluoro-5-(trifluoromethyl)phenyl]carbamoyl}amino)benzyl]amino}-1H-pyrazole-3-carboxamide trifluoroacetate;
4-{[3-({[4-(trifluoromethoxy)phenyl]carbamoyl}amino)benzyl]amino}-1H-pyrazole-3-carboxamide trifluoroacetate;
methyl 3-{[(3-{[(3-carbamoyl-1 H-pyrazol-4-yl)amino]methyl}phenyl)carbamoyl]-aminolbenzoate trifluoroacetate;
4-{[3-({[4-(trifluoromethyl)phenyl]carbamoyl}amino)benzyl]amino}-1H-pyrazole-3-carboxamide trifluoroacetate;
4-{[3-({[3-(trifluoromethyl)phenyl]carbamoyl}amino)benzyl]amino}-1H-pyrazole-3-carboxamide trifluoroacetate;
4-{[3-({[2-(trifluoromethyl)phenyl]carbamoyl}amino)benzyl]amino}-1H-pyrazole-3-carboxamide trifluoroacetate;
4-[((3-{[(3,5-dimethoxyphenyl)carbamoyl]amino}benzyl)amino]-1H-pyrazole-3-carboxamide trifluoroacetate;
4-[((3-{[(3-methylphenyl)carbamoyl]amino}benzyl)amino]-1H-pyrazole-3-carboxamide trifluoroacetate;
4-[((3-{[(4-methoxyphenyl)carbamoyl]amino}benzyl)amino]-1H-pyrazole-3-carboxamide trifluoroacetate;
4-[((3-{[(4-fluorophenyl)carbamoyl]amino}benzyl)amino]-1H-pyrazole-3-carboxamide trifluoroacetate;
4-{[3-({[4-chloro-3-(trifluoromethyl)phenyl]carbamoyl}amino)benzyl]amino}-1H-pyrazole-3-carboxamide trifluoroacetate;
4-{[3-({[4-(difluoromethoxy)phenyl]carbamoyl}amino)benzyl]amino}-1H-pyrazole-3-carboxamide trifluoroacetate;
4-{[3-({[2-chloro-4-(trifluoromethyl)phenyl]carbamoyl}amino)benzyl]amino}-1H-pyrazole-3-carboxamide trifluoroacetate;
4-[((3-{[(4-methylphenyl)carbamoyl]amino}benzyl)amino]-1H-pyrazole-3-carboxamide trifluoroacetate;
4-[((3-{[(2,5-dimethylphenyl)carbamoyl]amino}benzyl)amino]-1H-pyrazole-3-carboxamide trifluoroacetate;
4-[((3-{[(3,4-dimethylphenyl)carbamoyl]amino}benzyl)amino]-1H-pyrazole-3-carboxamide trifluoroacetate;
4-[((3-{[(2-methylphenyl)carbamoyl]amino}benzyl)amino]-1H-pyrazole-3-carboxamide trifluoroacetate;
4-[((3-{[(3-ethylphenyl)carbamoyl]amino}benzyl)amino]-1H-pyrazole-3-carboxamide trifluoroacetate;
4-{[3-({[3,5-bis(trifluoromethyl)phenyl]carbamoyl}amino)benzyl]amino}-1H-pyrazole-3-carboxamide trifluoroacetate;
4-[((3-{[(3-fluorophenyl)carbamoyl]amino}benzyl)amino]-1H-pyrazole-3-carboxamide trifluoroacetate;
4-[((3-{[(2-methoxy-5-methylphenyl)carbamoyl]amino}benzyl)amino]-1H-pyrazole-3-carboxamide trifluoroacetate;
4-[((3-{[(2,5-dimethoxyphenyl)carbamoyl]amino}benzyl)amino]-1H-pyrazole-3-carboxamide trifluoroacetate;
4-{[3-({[3-chloro-4-(difluoromethoxyphenyl]carbamoyl}amino)benzyl]amino}-1H-pyrazole-3-carboxamide trifluoroacetate;
4-[((3-{[(2,5-difluorophenyl)carbamoyl]amino}benzyl)amino]-1H-pyrazole-3-carboxamide trifluoroacetate;
4-{[3-({[4-methyl-3-(trifluoromethyl)phenyl]carbamoyl}amino)benzyl]amino}-1H-pyrazole-3-carboxamide trifluoroacetate;
methyl 4-{[3-({[2-fluoro-5-(trifluoromethyl)phenyl]carbamoyl}amino)benzyl]amino}-1H-pyrazole-3-carboxylate trifluoroacetate.

16. Product according to any one of the preceding claims, **characterized in that** it is:
4-((1-{3-[3-(2-fluoro-5-trifluoromethylphenyl)ureido]phenylethylamino}-1H-pyrazole-3-carboxamide trifluoroacetate;
4-({3-[3-(2-fluoro-5-trifluoromethylphenyl)ureido]benzyl}methylamino)-1H-pyrazole-3-carboxamide;
4-((ethyl-{3-[3-(2-fluoro-5-trifluoromethylphenyl)ureido]benzyl}amino)-1H-pyrazole-3-carboxamide;
4-({3-[3-(2-chloro-5-trifluoromethylphenyl)ureido]benzyl}methylamino)-1H-pyrazole-3-carboxamide;
4-{3-[3-(4-trifluoromethylpyrid-2-yl)ureido]benzylamino}-1H-pyrazole-3-carboxamide;
4-{3-[3-(4-methoxypyrid-2-yl)ureido]benzylamino}-1H-pyrazole-3-carboxamide;
4-{[3-({[3-chloro-4-fluorophenyl]carbamoyl}amino)benzyl]amino}-1H-pyrazole-3-carboxamide;
4-{[3-({[3,4-dichlorophenyl]carbamoyl}amino)benzyl]amino}-1H-pyrazole-3-carboxamide;
4-{[3-({[3-chloro-5-trifluoromethylphenyl]carbamoyl}amino)benzyl]amino}-1H-pyrazole-3-carboxamide;
4-{[3-({[3-trimethylsilyl-4-fluorophenyl]carbamoyl}amino)benzyl]amino}-1H-pyrazole-3-carboxamide;
4-{[3-({[3-trifluoromethoxyphenyl]carbamoyl}amino)benzyl]amino}-1H-pyrazole-3-carboxamide;
4-{[3-({[3-trifluoromethyl-4-chlorophenyl]carbamoyl}amino)benzyl]amino}-1H-pyrazole-3-carboxamide;
4-{[3-({[2-chloro-5-trifluoromethylphenyl]carbamoyl}amino)benzyl]amino}-1H-pyrazole-3-carboxamide;
4-{[3-({[3-trifluoromethylsulfanylphenyl]carbamoyl}amino)benzyl]amino}-1H-pyrazole-3-carboxamide;
4-{[3-({[3-isopropylphenyl]carbamoyl}amino)benzyl]amino}-1H-pyrazole-3-carboxamide;
4-{[3-({[3-isopropyl-4-fluorophenyl]carbamoyl}amino)benzyl]amino}-1H-pyrazole-3-carboxamide;
4-{[3-({[3-pentafluorosulfanylphenyl]carbamoyl}amino)benzyl]amino}-1H-pyrazole-3-carboxamide;
4-{[3-({[2-methoxy-5-tert-butylphenyl]carbamoyl}amino)benzyl]amino}-1H-pyrazole-3-carboxamide;
4-{[3-({[4-isopropylphenyl]carbamoyl}amino)benzyl]amino}-1H-pyrazole-3-carboxamide;
4-{[3-({[2-chloro-4-isopropylphenyl]carbamoyl}amino)benzyl]amino}-1H-pyrazole-3-carboxamide;
4-{[3-({[2-fluoro-5-methylphenyl]carbamoyl}amino)benzyl]amino}-1H-pyrazole-3-carboxamide;
4-{[3-({[2-fluoro-4-trifluoromethylphenyl]carbamoyl}amino)benzyl]amino}-1H-pyrazole-3-carboxamide;
4-{[3-({[2-fluoro-4-methylphenyl]carbamoyl}amino)benzyl]amino}-1H-pyrazole-3-carboxamide;
4-{[3-({[2-chloro-4-methylphenyl]carbamoyl}amino)benzyl]amino}-1H-pyrazole-3-carboxamide;
4-{[3-({[2-chloro-5-methylphenyl]carbamoyl}amino)benzyl]amino}-1H-pyrazole-3-carboxamide;
(RS)-4-(1-{3-[3-(2-chloro-4-trifluoromethylphenyl)ureido]phenyl}ethylamino)-1H-pyrazole-3-carboxamide.

17. Product according to any one of the preceding claims, **characterized in that** it is:
1) in non-chiral form, or
2) in racemic form, or
3) enriched in one stereoisomer, or
4) enriched in one enantiomer;
and **in that** it is optionally salified.

18. Pharmaceutical composition comprising a product according to any one of Claims 1 to 17, in combination with a pharmaceutically acceptable excipient.

19. Use of a product according to any one of Claims 1 to 17, for the manufacture of a medicament that is useful for treating a pathological condition.

20. Use according to Claim 19, **characterized in that** the pathological condition is cancer.

21. Process for preparing the products of general formula (Ib) below: 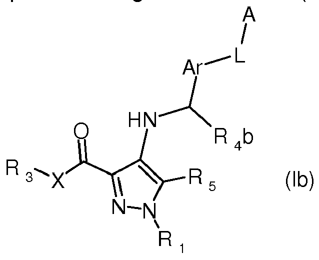 in which R₁, R₃, R_{4b}, R₅, X, Ar, L and A are as defined in Claim 1, and R₄ₐ is H, **characterized in that** a product of general formula (II) below: 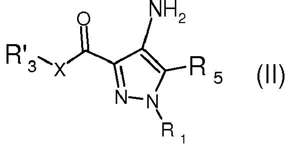 in which R'₃ is R₃ or a precursor of R₃, and X, R₁, R₃ and R₅ are as defined in Claim 1, reacts with a product of formula (III) below: 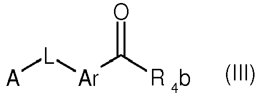 in which R_{4b}, Ar, L and A are as defined in Claim 1, to give the product of general formula (Ib).

22. Process for preparing the products of general formula (I) below: 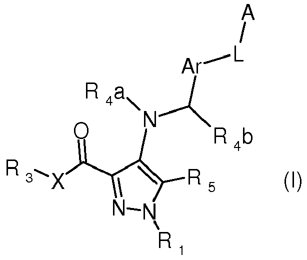 in which R₁, R₃, R₄ₐ, R_{4b}, R₅, X, Ar and A are as defined in Claim 1, and L is NHCONH, **characterized in that** a product of general formula (VIII) below: 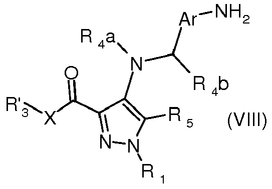 in which R'₃ is R₃ or a precursor of R₃, and X, Ar, R₁, R₃, R₄ₐ, R_{4b} and R₅ are as defined in Claim 1, reacts with a product of formula (VII) below: 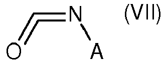 in which A is as defined in Claim 1, to give the product of general formula (I') below 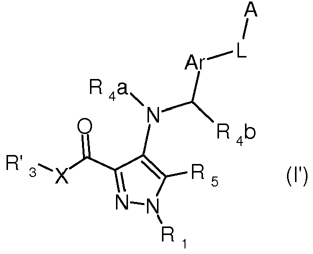 in which the precursor R'₃ is transformed into R₃ in order to obtain the product of general formula (I).

23. As intermediate products, the products of general formula (VIII), for which Ar, R'₃, X, R₁, R₄ₐ, R_{4b} and R₅ are as defined in Claim 22.
